# EUROPEAN PATENT SPECIFICATION

(11) **EP 2 431 353 B1**
(45) Date of publication and mention of the grant of the patent: **20.01.2016**
(21) Application number: 11181333.3
(22) Date of filing: 15.09.2011
(51) Int. Cl.: C07C 209/26, C07C 213/02

(54) **Process for preparing amine compound**
Verfahren zur Herstellung von Amin Verbindung
Procédé de préparation d'un composé amine

(30) Priority: 15.09.2010 JP 2010207142
(43) Date of publication of application: 21.03.2012
(73) Proprietor: Kanto Kagaku Kabushiki Kaisha, Chuo-ku Tokyo (JP)
(72) Inventor: Watanabe, Masahito, Soka-shi, Saitama (JP); Tanaka, Kouichi, Soka-shi, Saitama (JP); Miki, Takashi, Soka-shi, Saitama (JP); Murata, Kunihiko, Soka-shi, Saitama (JP)
(74) Representative: Lecca, Patricia S.

(56) References cited:
- EP-A1- 2 172 443
- EP-A1- 2 228 377
- WO-A1-2006/004093
- WO-A1-2009/080511
- WO-A1-2009/080512

## Description

### Technical Field

The present invention relates to a practical process for preparing an amine compound by means of diastereoselective and reductive amination reaction of a carbonyl compound, and a practical process for preparing an amine compound by means of diastereoselective reduction or hydrogenation of an imine compound or enamine compound, using a nitrogen-containing organometallic complex as a catalyst.

### Background Art

A process for preparing an amine compound by means of reaction of ammonia or a primary or secondary amine compound with a carbonyl compound has been known as a reductive amination reaction, which is one of the standard processes for preparing amine compounds. As the reductive amination reaction, except for enantioselective methods using an asymmetric catalyst, the following methods are generally carried out: methods by hydrogenation reaction using heterogenous catalysts such as Raney Ni, Raney Co, Pd/activated carbon, and Pt/activated carbon, as well as methods using boron reactants such as NaBH₃CN (J. Am. Chem. Soc. 1971, 93, 2897) and NaBH(OAc)₃ (J. Org. Chem. 1996, 61, 3849). Under the presence of these heterogenous catalysts and boron reactants, when a carbonyl compound or amine compound having a chiral carbon or achiral carbon is used as the reactive substrate, it has been known that a reactive intermediate, i.e., an imine compound or enamine compound, is diastereoselectively reduced.

For example, in the synthesis of 4-tert-butylcyclohexylamine by reductive amination reaction of 4-tert-butylcyclohexanone and ammonia, as described in Non-patent Literature 1 and Patent Literature 1, corresponding amines can be obtained with a diastereoselectivity of cis:trans = 90 : 10 by hydrogenation reaction using a non-homogenous catalyst such as rhodium catalyst or palladium catalyst. When 4-tert-butylcyclohexanone is reacted with a primary amine and the obtained imine is subjected to hydrogenation reaction using a heterogenous catalyst or to reductive reaction using a boron reactant as described in Patent Literature 2, Non-patent Literature 2, Non-patent Literature 3 and Non-patent Literature 4, corresponding amines can be obtained with a selectivity of cis:trans = 99:1 at the maximum.

Furthermore, as a method to obtain optically active amines using diastereoselective reaction, as described in Non-patent Literature 5, the following method has been known: after diastereoselectively obtaining a corresponding amine by reductive amination reaction of a carbonyl compound with an optically active α-methylbenzylamine under the presence of a heterogenous catalyst (Raney Ni, Pd/activated carbon, Pt/activated carbon, Ru/activated carbon, or Rh/activated carbon) and a Lewis acid such as Ti(O-i-Pr)₄ etc., an optically active primary amine is obtained by de-benzylation reaction.

However, with the methods described in Non-patent Literature 1, Non-patent Literature 5, Patent Literature 1, and Patent Literature 2 using a heterogenous catalyst, when the substrate has a site of carbon-carbon multiple bond and a functional group such as cyano group and nitro group, they are simultaneously hydrogenated, so that the methods cannot be applied to such substrates having functional groups. In addition, with the methods described in Non-patent Literature 1 and Patent Literature 1, there are problems of lower diastereoselectivity compared to those of Non-patent Literature 3 and 4.

The methods using boron reactants described in Non-patent Literatures 2-4 have excellent operability and safety because no pressure-resistant reactor is required, and have no problem in terms of functional selectivity which is the problem in heterogeneous catalysts; however, they are not superior in terms of economic efficiency and environmental aspect because they are not a catalyzed reaction. In addition, in Non-patent Literatures 3 and 4, although high diastereoselectivity is obtained, an imine must be prepared beforehand, and deprotection of the obtained amine after reduction of the imine is required; and an expensive boron reactant must be used to obtain high diastereoselectivity. Thus, the methods are not a practical preparation process.

In Patent Literature 3, a process for preparing optically active alcohols by asymmetric reduction of carbonyl compounds using an organometallic complex as a catalyst has been described; however, whether or not reductive amination can be catalyzed by such catalyst has not been reported. In addition, in Patent Literature 4, a method to enantioselectively obtain amine compounds using an organometallic complex in reductive amination reaction has been described; however, sufficient enantioselectivity cannot be achieved.

Patent Literature 5 described a method to enantioselectively obtain amine compounds using an organometallic complex having a chiral prolinamide compound as a ligand, in which nitrogen atom of pyrrolidine in the prolinamide has a hydrogen.

Patent Literature 6 described a method for manufacturing amines by converting a carbonyl compound with an amine in the presence of a heterogeneous imination catalysts containing copper, a hydrogenation catalyst and hydrogen.

Patent Literature 7 described diastereoselective conversion of chiral imines in the presence of hydrogen and a heterogeneous catalyst containing copper.

As described above, development of a generalized practical process for preparing amine compounds through highly-diastereoselective reductive amination reaction, which exhibits high activity and excellent functional selectivity, has conventionally been desired.

### [Citation List]

### [Patent Literature]

[Patent Literature 1] EP 0669314 A1
[Patent Literature 2] WO99/047987
[Patent Literature 3] WO02/010101
[Patent Literature 4] WO2006/004093
[Patent Literature 5] EP 2172443 A1
[Patent Literature 6] WO2009/080512 A1
[Patent Literature 7] WO2009/080511 A1

### [Non-patent Literature]

[Non-patent Literature 1] Journal of the Chemical Society of Japan, 1989, p. 641-647
[Non-patent Literature 2] Tetrahedron Letters (1996), 37(23), 3977-3980.
[Non-patent Literature 3] Tetrahedron Letters (1984), 25(7), 695-8.
[Non-patent Literature 4] Tetrahedron Letters (1981), 22(36), 3447-50.
[Non-patent Literature 5] Adv. Synth. Catal. 2006, 348, 1289-1299.

### Summary of Invention

### Technical Problem

An object of the present invention is to provide a generalized practical process for preparing amine compounds through highly-diastereoselective reductive amination reaction.

### Solution to Problem

The present inventors have extensively investigated a process for preparing amine compounds by reductive amination reaction of carbonyl compound and amine compound, and found that ruthenium, rhodium and iridium complexes having a nitrogen-containing ligand enable highly-diastereoselective preparation of amine compounds in the reductive amination reaction; after further research, the inventors have achieved the present invention.

Namely, the present invention relates to the following.
(a) A process for preparing an amine compound, characterized in that an imine compound or an enamine compound is diastereoselectively reduced using hydrogen gas or an organic or inorganic hydrogen-donating compound, under the presence of an organometallic compound of general formula (1): 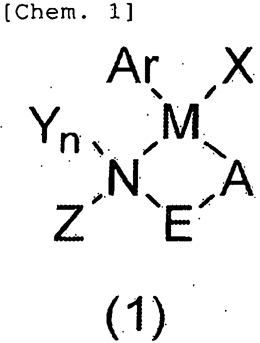 wherein in general formula (1), Ar is a cyclopentadienyl group or an aromatic compound, in which one or more hydrogen atoms may be substituted by a substituent W, and W denotes a saturated or unsaturated C1-10 hydrocarbon group, anarylgroup, a heterocyclyl group, an alkoxy group, a fluoroalkyl group, an acyl group, an ester group, a hydroxyl group, an amino group, an amide group, a carboxyl group, a sulfonyl group, a nitro group, a cyano group, a sulfenyl group, a sulfo group, a mercapto group, a phosphino group, a silyl group or a halogen group, M is ruthenium, rhodium, or iridium, X is a hydride group or an anionic group, E denotes a link group which is a saturated or unsaturated C1-6 hydrocarbon group in which one or more hydrogen atoms may be substituted by a substituent W, or which is an arylene group in which one or more hydrogen atoms may be substituted by C1-10 alkyl group, cycloalkyl group, aryl group, alkenyl group, acyl group, halogen group, ester group, amino group, amide group, carboxyl group, hydroxyl group, nitro group, cyano group, sulfenyl group, sulfo group, or thiol group (wherein one or more hydrogen atoms in each of said substituents may further be substituted by W), A is an amide group having a binding mode of "M-NR¹C(O) -E, " or an oxygen atom, wherein when A is an oxygen atom, E is an arylene group in which one or more hydrogen atoms may be substituted by a substituent W, R¹ denotes a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W (wherein one or more hydrogen atoms in the substituent W may further be substituted by a substituent W), Y and Z are identical or mutually different, and denote a C1-10 alkyl group, a cycloalkyl group, an aryl group, a heterocyclyl group, an alkenyl group, or a silyl group, in which one or more hydrogen atoms may be substituted by a substituent W, wherein "Y and Z," "Z and E," "Y and E," or "Y-Z-E" may be bound to form a ring, and n denotes an integer of 0 or 1, wherein when n=0, the bond between N-Z or N-E is a double bond.
(b) The process for preparing an amine compound according to (a), characterized in that a part of formula (1) expressed by general formula (2) below: 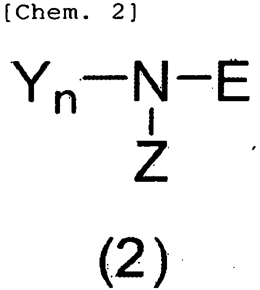 wherein in general formula (2), n=0 and N, E and Z have the same meanings as defined above, is a group comprising a nitrogen-containing cyclic group.
(c) The process for preparing an amine compound according to (a) or (b), characterized in that the organometallic compound of formula (1) is a compound of general formula (3): 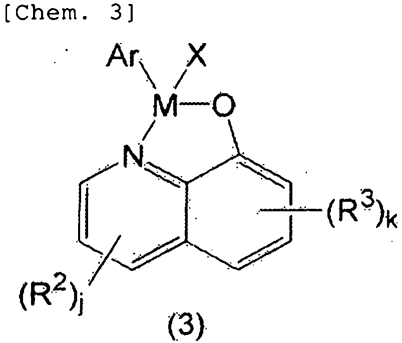 wherein in general formula (3), Ar, M and X have the same meanings as defined in (a), R² and R³ are mutually identical or mutually different, and denote a hydrogen atom, a halogen atom, a nitro group, a cyano group, a hydroxyl group, a sulfo group, a mercapto group, a carboxyl group, or the following groups in which one or more hydrogen atoms may be substituted by a substituent W: a C1-10 alkyl group, a cycloalkyl group, an aryl group, a heterocyclyl group, an alkenyl group, an alkynyl group, an alkoxy group, an ester group, a fluoroalkyl group, an acyl group, a sulfonyl group, an amino group, an amide group, a phosphino group, a sulfenyl group, or a silyl group, W has the same meaning as described in (a.), j and k are, independently of each other, an integer from 0 to 3, wherein one or more carbon atoms, which is not bonded to R² or R³, in the quinoline ring may be replaced by a nitrogen atom, and when j and/or k is(are) 1 or more, R² and R³ may be linked to each other to form a ring, and when j and/or k is (are) 2 or more, two or more of R² and/or R³ may be mutually linked to form a ring,
   or one or more organometallic compounds selected from the group consisting of the organometallic compound of general formula (4) below: 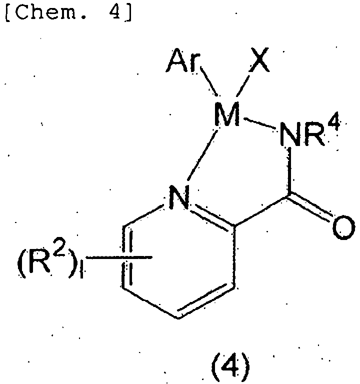 wherein in general formula (4), Ar, M and X have the same meanings as defined (a), R² has the same meaning as the above R² and R³, R⁴ denotes a hydrogen atom, a C1-10 alkyl group, a cycloalkyl group, an aryl group, a heterocyclyl, group" an alkenyl group, an alkynyl group, an ester group, an acyl group, an amide group, a phosphino group, a sulfenyl group, a sulfenyl group, a sulfonyl group or a silyl group, in which one or more hydrogen atoms may be substituted by a substituent W, W has the same meaning as described in (a), 1 denotes an integer from 0 to 4, wherein one or more carbon atoms, which is not bonded to R², in the pyridine ring may be replaced by a nitrogen atom, and wherein when 1 is 2 or more, two or more of R² may be linked each other to form a ring,
   and general formula (5) below: 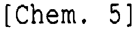 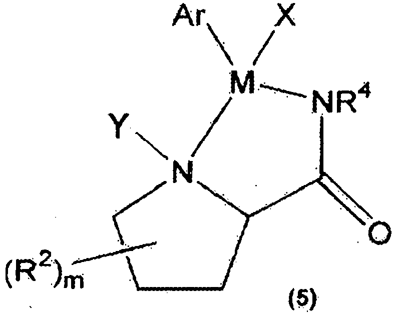 wherein in general formula (5), Ar, M, X and Y have the same meanings as defined in (a), R² has the same meaning as the above R² and R³, R⁴ denotes a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W, W has the same meaning as described in (a), m denotes an integer from 0 to 7, wherein one or more carbon atoms in the pyrrolidine ring may be replaced by a nitrogen atom, and wherein when m is 2 or more, two or more of R² may be linked each other to form a ring.
(d) A process for preparing an amine compound, characterized in that an imine compound or an enamine compound is diastereoselectively reduced using hydrogen gas or an organic or inorganic hydrogen-donating compound, under the presence of an organometallic compound of general formula (6): 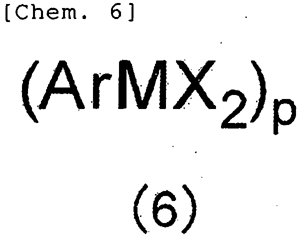 wherein in general formula (6), Ar, M and X have the same meanings as defined in (a), and p denotes an integer of 2 or more, and an organic compound of general formula (7): 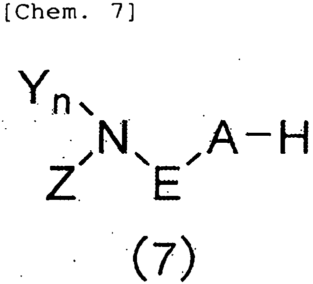 wherein in general formula (7), A, E, Y, Z and n have the same meanings as defined in (a).
(e) The process for preparing an amine compound according to any one of (a) - (d), characterized in that a carbonyl compound and an amine compound are mixed, and the imine compound or enamine compound generated in this system is diastereoselectively reduced.
(f) The process for preparing an amine compound according to any one of (a)-(e), characterized in that M in formula (1) and formula (6) is rhodium or iridium.
(g) The process for preparing an amine compound according to any one of Claims (a)-(f), characterized in that the organic or inorganic hydrogen-donating compound is formic acid or formate.
(h) The process for preparing an amine compound according to any one of (a)-(g), characterized in that the amine compound obtained from the reaction is a cyclic amine compound or a cyclic diamine compound.

### Advantageous Effects of Invention

The present invention provides a practical process for preparing amine compounds by highly-diastereoselective reductive amination reaction of carbonyl compound and amine compound, thereby enabling highly efficient preparation of amine compounds.

### Brief Description of the Figures

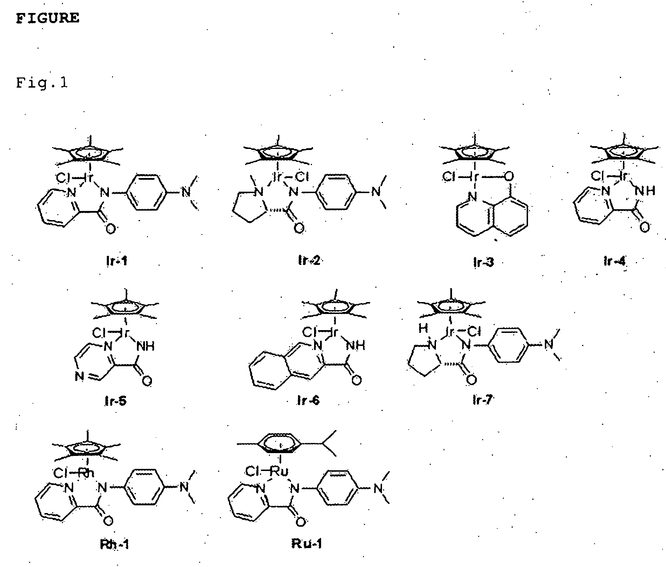

[Fig. 1] Figure 1 shows representative organometallic compounds of the present invention.

### Description of Embodiments

Hereinafter, the present invention is described in detail. The nitrogen-containing organometallic complex used in the present invention is represented by general formula (1).

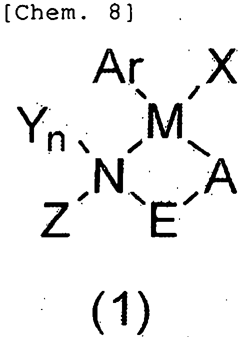

Ar is, typically, a cyclopentadienyl group or an aromatic compound in which one or more hydrogen atoms may be substituted; specific examples of Ar include, but are not limited to, unsubstituted benzene, toluene, o-, m- and p-xylene, o-, m-and p-cymene, 1,2,3-, 1,2,4- and 1,3,5-trimethylbenzene, 1,2,4,5-tetramethylbenzene, 1,2,3,4-tetramethylbenzene, 1,3,4,5-tetramethylbenzene, pentamethylbenzene, and benzene having an alkyl group such as hexamethylbenzene, benzene having an unsaturated hydrocarbon group such as benzyl, vinyl, and allyl, benzene having a heteroatom such as hydroxyl group, alkoxy group, ester group, amino group and halogen group. The number of substituents in a benzene ring is any number from 1 to 6, and substituents may be at any positions. From the viewpoint of easiness in the synthesis of complex, Ar is preferably p-cymene, 1,3,5-trimethylbenzene, and hexamethylbenzene.

Examples of cyclopentadienyl group that may have a substituent include, but are not limited to, cyclopentadienyl group, methylcyclopentadienyl group, ethylcyclopentadienyl group, isopropylcyclopentadienyl group, phenylcyclopentadienyl group, benzylcyclopentadienyl group, 1,2-dimethylcyclopentadienyl group, 1,3-dimethylcyclopentadienyl group, 1,2,3-trimethylcyclopentadienyl group, 1,2,4-trimethylcyclopentadienyl group, 1,2,3,4-tetramethylcyclopentadienyl group, and 1,2,3,4,5-pentamethylcyclopentadienyl group (Cp*). From the viewpoint of easiness in the synthesis of complex, it is preferably a 1,2,3,4,5-pentamethylcyclopentadienyl group (Cp*).

The substituent W is a saturated or unsaturated C1-10 hydrocarbon group, aryl group, heterocyclyl group, alkoxy group, fluoroalkyl group, acyl group, ester group, hydroxyl group, aminogroup, amide group, carboxyl group, sulfonyl group, nitro group, cyano group, sulfenyl group, sulfo group, mercapto group, phosphino group, silyl group or halogen group; in particular, it is a saturated or unsaturated C1-10 hydrocarbon group, aryl group, heterocyclyl group, alkoxy group, acyl group, ester group, hydroxyl group, amino group, sulfonyl group, silyl group or halogen group.

Specific examples of W include, but are not limited to, methyl group, ethyl group, n-propyl group, i-propyl group, n-butyl group, i-butyl group, pentyl group, hexyl group, cyclohexylene group, ethenyl group, propenyl group, butenyl group, phenyl group, toluyl group, naphthyl group, pyridyl group, furanyl group, methoxy group, ethoxy group, propoxy group, acetyl group, propanoyl group, cyclohexanecarbonyl group, benzoyl group, methoxycarbonyl group, ethoxycarbonyl group, hydroxyl group, methylamino group, ethylamino group, dimethylamino group, methanesulfonyl group, ethanesulfonyl group, methylsilyl-group, dimethylsilyl group, trimethylsilyl group, fluoro group, chloro group, and trifluoromethyl group, etc. From the viewpoint of easiness in the synthesis of complex, it is preferably a saturated or unsaturated hydrocarbon group, and more preferably a methyl group and i-propyl group.
M in general formula (1) is ruthenium, rhodium or iridium. From the viewpoint of high level of catalyst activity, it is preferably rhodium and iridium.

X in general formula (1) is a hydride group or anionic group. Specific examples of X include, but are not limited to, hydride group, hydroxy group, alkoxy group, cross-linked oxo group, fluorine gr.oup, chlorine group, bromine group, iodine group, tetrafluoroborate group, tetrahydroborate group, tetrakis(pentafluorophenyl)borate group, tetrakis [3', 5'-bis (trifluoromethyl) phenyl] borate group, hexafluorophosphate group, hexafluoroantimonate group, hexachloroantimonate group, hexafluoroarsenate group, perchlorate group, acetoxy group, benzoyloxy group, (2',6'-dihydroxybenzoyl)oxy group, (2',5'-dihydroxybenzoyl)oxy group, (3'-aminobenzoyl)oxy group, (2',6'-dimethoxybenzoyl)oxy group, (2',4',6'-triisopropylbenzoyl)oxy group, 1-naphthalenecarboxylic acid group, 2-naphthalenecarboxylic acid group, trifluoroacetoxy group, trifluoromethanesulfonimide group, nitromethyl group, nitroethyl group, toluenesulfonate group, methanesulfonate group, ethanesulfonate group, n-propanesulfonate group, isopropanesulfonate group, n-butanesulfonate group, fluorosulfonate group, fluoromethanesulfonate group, difluoromethanesulfonate group, trifluoromethanesulfonate group, and pentafluoroethanesulfonate group, etc. From the viewpoint of easiness in the synthesis of complex, it is preferably a chlorine group, bromine group, iodine group, and trifluoromethanesulfonate group.

Furthermore, in addition to an anionic group X, a coordinating neutral molecule may be coordinated to M. Specific examples of the neutral molecule coordinated to M include water; alcohols such as methanol, ethanol, n-propanol, isopropanol and 2-methoxyethanol; ethers such as tetrahydrofurane, diethyl ether, diisopropyl ether, and methyl-tert-butyl ether; N,N'-dimethyl formamide, N,N'-dimethyl acetoamide, N-methyl-2-pyrrolidone, dimethylsulfoxide, acetone, chloroform, acetonitrile, benzonitrile, etc.

The link group E is a saturated, or unsaturated C1-6 hydrocarbon group or an arylene group, in which one or more hydrogen atoms may be substituted. Specific examples of E include, but are not limited to, methylene group, ethylene group, propylene group, cyclohexylene group, vinylene group, propynylene group, butenylene group, phenylene group, and naphthalenylene group. From the viewpoints of high catalyst activity and easiness in the synthesis of complex, it is preferably a methylene group, ethylene group, phenylene group, and naphthalenylene group, and more preferably a methylene group and phenylene group.

When E is an arylene group, examples of the substituents for one or more hydrogen atoms typically include C1-10 alkyl group, cycloalkyl group, aryl group, alkenyl group, acyl group, halogen group, ester group, amino group, amide group, carboxyl group, hydroxyl group, alkoxy group, nitro group, cyano group, phosphino group, sulfonyl group, sulfenyl group, sulfo group, thiol group, or silyl group, and in particular, C1-10 alkyl group, cycloalkyl group, aryl group, alkenyl group, or acyl group. One or more hydrogen atoms in these substituents may further be substituted by a substituent W.

A in general formula (1) is an amide group having a binding mode of "M-NR¹C(O) -E, " or an oxygen atom. R¹ is a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W. One or more hydrogen atoms of the substituent W in these groups may further be substituted by a substituent W.

Specific examples of R¹ include, but are not limited to, hydrogen atom, methyl group, ethyl group, n-propyl group, i-propyl group, tert-butyl group, isobutyl group, benzyl group, cyclohexyl group, phenyl group, pyridyl group, vinyl group, ethynyl group, group having an ester bond, acetyl group, benzoyl group, methanesulfonyl group, ethanesulfonyl group, p-toluenesulfonyl group, trifluoromethansulfonyl group, methylsilyl group, dimethylsilyl group, trimethylsilyl group, etc. From the viewpoints of high catalyst activity and high reaction yield, it is preferably a hydrogen atom, methyl group, isopropyl group, tert-butyl group, isobutyl group, benzyl group, phenyl group, 4-methoxyphenyl group, and 4-(dimethylamino)phenyl group. When A is an oxygen atom, E is limited to an arylene group wherein one or more hydrogen atoms may be substituted by a substituent W.

Y and Z in general formula (1) are, typically and independently of each other, an alkyl group, a cycloalkyl group, a heterocyclyl group, a silyl group, an aryl group, or an alkenyl group, in which one or more hydrogen atoms may be substituted by a substituent W. Specific examples of Y and Z include methyl group, ethyl group, cyclohexyl group, phenyl group, pyridyl group, vinyl group, and trimethylsilyl group, but are not limited thereto. From the viewpoint of easiness in the synthesis of complex, they are preferably a methyl group, ethyl group and phenyl group. Y, Z and E may be mutually bound to form a ring. Specific examples include, but are not limited to, cases wherein Z and E are bound to form a piperidyl group, pyridyl group, or pyrrolidyl group. When n denotes 0, N-Z or N-E is a double bond.

By using an organometallic complex having the structure of the above formula (1) as a catalyst for reductive amination reaction of an imine compound or enamine compound, under the presence of hydrogen gas or a hydrogen-donating organic or inorganic compound, the imine compound or enamine compound can be diastereoselectively reduced. Mechanism of this diastereoselective reduction has not yet been clarified, but one cause is considered to be related to the size of the molecular structure of said organometallic complex. This organometallic complex is a sterically bulky molecule, so that upon acting as a catalyst for reductive amination, reduction tends to occur from the direction with less steric hindrance against the reaction site of the imine structure.

The part in the catalyst of formula (1), the structure of which is expressed by general formula (2) below: 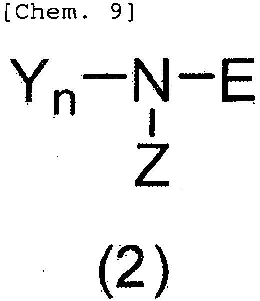 is preferably a group comprising a nitrogen-containing cyclic group, from the viewpoints of high catalyst activity and diastereoselectivity. When n is 0, said group is a nitrogen-containing aromatic ring group, and N in general formula (2) is a hetero nitrogen atom in the nitrogen-containing aromatic ring structure, so that Z and E are bound to form a ring structure. Examples of the group comprising the nitrogen-containing aromatic ring structure include, not only a nitrogen-containing six-membered monocyclic aromatic ring such as pyridine ring, pyridazine ring, pyrazine ring, pyrimidine ring, triazine ring or tetrazine ring, but also a polycyclic aromatic ring comprising a nitrogen-containing six-membered aromatic ring structure, such as quinoline ring, isoquinoline ring, quinazoline ring, quinoxalinering, acridinering, cinnolinering, phthalazinering, naphthyridine ring. In addition, such group may be any group comprising a nitrogen-containing aromatic ring structure, and includes, for example, 5,6,7,8-tetrahydroquinolyl group. Examples of nitrogen-containing 5-membered ring include pyrazole ring, isooxazole ring, isothiazole ring, imidazole ring, oxazole ring, thiazole ring, 1,2,3-triazole ring, 1,2,4-triazole ring, tetrazole ring, etc.

When n=1, said nitrogen-containing cyclic group becomes a non-aromatic ring, and N becomes a nitrogen heteroatom in the nitrogen-containing ring, so that a ring structure is formed by binding Z and E. Examples of the group containing such non-aromatic nitrogen-containing ring structure include, a 4-membered nitrogen-containing ring such as azetidine ring, a 5-membered nitrogen-containing ring or a group containing said ring, such as pyrrolidine ring, pyrroline ring, imidazolidine ring, imidazoline ring, pyrazolidine ring, pyrazoline ring, oxazoline ring, indoline ring, and isoindoline ring, a 6-membered nitrogen-containing ring or a group containing said rind, such as piperidine ring, piperazine ring, morpholine ring, as well as quinuclidine ring.

From the viewpoints of high catalyst activity and high diastereoselectivity, a compound of general formula (3): 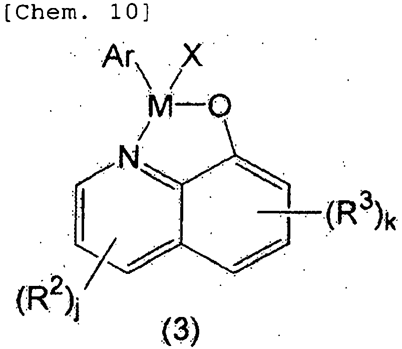 is preferred. In this formula, R² and R³ are, independently of each other, a hydrogen atom, a halogen atom, or a C1-10 alkyl group, a cycloalkyl group, an aryl group, a heterocyclyl group, an alkenyl group, an alkynyl group, an alkoxy group, an ester group, a fluoroalkyl group, an acyl group, a sulfonyl group, a nitro group, a cyano group, a hydroxy group, a sulfo group, a mercapto group, an amino group, an amide group, a carboxyl group, a phosphino group, a sulfenyl group, or a silyl group, in which one or more hydrogen atoms may:be substituted by a substituent W; in particular, they are a hydrogen atom, a halogen atom, or a C1-10 alkyl group, a cycloalkyl group, an aryl group, a heterocyclyl group, an alkenyl group, an alkynyl group, an alkoxy group, an ester group, an acyl group, a sulfonyl group, a hydroxyl group, or a silyl group, in which one or more hydrogen atoms may be substituted by a substituent W.

A plurality of R² and R³ may be introduced, and the numbers of substitution j and k are, independently of each other, an integer from 0 to 3, wherein when j is 2 or more, two or more of R² may be linked each other to form a ring such as phenanthridine ring or acridine ring. Furthermore, one or more carbon atoms, which are not bonded to R² or R³, in the quinoline ring structure may be replaced by a nitrogen atom to form, for example, naphthyridine ring, quinoxaline ring, cinnoline ring, quinazoline ring, phthalazine ring, etc.

Specific examples of R² and R³ include methyl group, ethyl group, cyclohexyl group, phenyl group, pyridyl group, vinyl group, ethynyl group, group having an ester bond, acetyl group, methanesulfonyl group, ethanesulfonyl group, p-tluenesulfonyl group, and trimethylsilyl group, but are not limited thereto. From the viewpoint of high catalyst activity, they are preferably a methyl group, ethyl group and propyl group.

In addition, from the viewpoints of high catalyst activity and high diastereo selectivity, a compound of general formula (4): 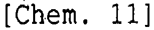 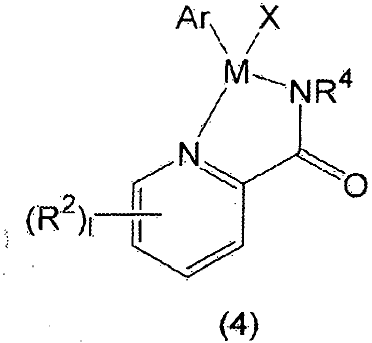 is preferred. In this formula, R² has the same meaning as described above, and its number of substitution 1 is an integer from 0 to 4. R⁴ is a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group, or a silyl group, in which one or more hydrogen atoms may be substituted by a substituent W.

Specific examples of R⁴ include, but are not limited to, for example, hydrogen atom, methyl group, ethyl group, n-propylgroup, isopropyl group, tert-butylgroup, isobutyl group, benzyl group, cyclohexylgroup, phenyl group, pyridylgroup, vinyl group, ethynyl group, group having an ester bond, acetyl group, benzoyl group, sulfonyl group, and silyl group. From the viewpoints of high catalyst activity and high reaction yield, it is preferably a hydrogen atom, methyl group, ethyl group, isopropyl group, tert-butyl group, isobutyl group, benzyl group, phenyl group, 4-methoxyphenyl group, and 4-(dimethylamino)phenyl group. A plurality of R² may be introduced, and the number of substitution 1 denotes an integer from 0 to 4, wherein when 1 is 2 or more, two or more of R² may be linked each other to form a ring such as quinoline ring, isoquinoline ring, etc. Furthermore, one or more carbon atoms, which are not bonded to R², in the pyridine ring structure may be replaced by a nitrogen atom to form, for example, a pyridazine ring, pyrazine ring, pyrimidine ring, and triazine ring, etc.

In addition, from the viewpoints of high catalyst activity and high diastereoselectivity, a compound of general formula (5) : 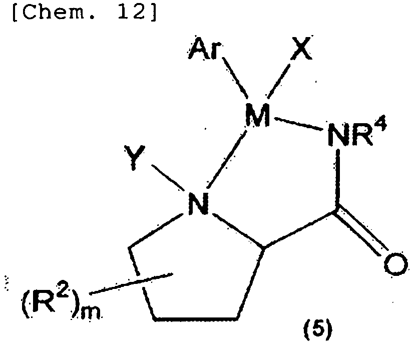 is preferred. In this formula, R² has the same meaning as described above, and its number of substitution m is an integer from 0 to 7. R⁴ is a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group, or a silyl group, in which one or more hydrogen atoms may be substituted by a substituent W.

Specific examples of R⁴ include, but are not limited to, for example, hydrogen atom, methyl group, ethyl group, n-propyl group, isopropyl group, tert-butyl group, isobutyl group, benzyl group, cyclohexylgroup, phenyl group, pyridylgroup, vinyl group, ethynyl group, group having an ester bond, acetyl group, sulfonyl group, and silyl group. From the viewpoints of high catalyst activity and high reaction yield, it is preferably a hydrogen atom, methyl group, ethyl group, isopropyl group, tert-butyl group, isobutyl group, benzyl group, phenyl group, 4-methoxyphenyl group, or 4-(dimethylamino)phenyl group. A plurality of R² may be introduced, and the number of substitution m denotes an integer from 0 to 7, wherein when m is 2 or more, two or more of R² may be linked each other to form a ring, such as indoline ring, isoindoline ring, etc. Furthermore, one or more carbon atoms in the pyrrolidine ring structure may be replaced by a nitrogen atom to form, for example, a imidazolidine ring, pyrazolidine ring, etc.

To use an organometallic complex as an asymmetric reduction catalyst as described above is publicly known. For example, in the above Patent Literatures 3 and 4, an asymmetric reduction reaction using an organometallic complex that has H as a substituent corresponding to Y in general formula (1) of this application is disclosed.
Patent Literature 3 discloses a process for preparing an optically active alcohol by asymmetric reduction of a carbonyl compound, but not a process for diastereoselectively preparing amine compounds.

Patent Literature 4 discloses a preparation method of tamsulosin, wherein in its one process, a step to obtain an enantiomer by asymmetric reduction of an imine compound is disclosed. However, in the asymmetric reduction reaction of imine compound using an organometallic complex in which the substituent corresponding to Y in general formula (1) of the present invention is a hydrogen atom, the enantioselectivity obtained is 30-40%ee, which is not sufficient, and optically active bodies with sufficient purity can be obtained only after further purification using re-crystallization.

In contrast, the organometallic complex used in the process of the present invention is particularly characterized by that the nitrogen atom of the hetero ring coordinated to a metal atom does not have a hydrogen atom, namely, that Y is not H. In the process of the present invention, by using an organometallic complex wherein Y is not H, a reductive amination reaction with extremely high reactivity and diastereoselectivity can be achieved compared to conventional organometallic complexes. In addition, when the organometallic complex wherein Y is H is reacted with a carbonyl compound under a reductive condition, the following reaction proceeds: the imine compound or enamine compound is rarely reduced, and the carbonyl compound is reduced to generate an alcohol; therefore, a one-pot reductive amination reaction wherein a carboxyl compound is mixed with an amine compound, then the imine compound or enamine compound generated in the system is diastereoselectively reduced, cannot be achieved. However, the present invention enables such one-pot reaction.

The reason that high diastereoselectivity can be obtained when Y is not H has not yet been clarified; however, because the organometallic complex of the present invention is sterically bulky, the complex is considered to be significantly affected by the steric environment of substituents in the imine compound or enamine compound to be reduced. In addition, the reason that reduction of carbonyl compound does not occur when Y is not H has not yet been clarified as well; however, this is considered to be because the organometallic complex of the present invention does not have an ability to reduce carbonyl compounds under an acidic condition under which imine or enamine compounds are easily produced.

Under the presence of the catalyst used in the present invention, using hydrogen gas or a hydrogen-donating organic or inorganic compound, it is possible to prepare an amine compound by diastereoselectively reducing an imine compound or enamine compound. Moreover, under the presence of the catalyst used in the present invention, using hydrogen gas or a hydrogen-donating organic or inorganic compound, it is possible to prepare an amine compound by mixing a carbonyl compound with an amine compound, then diastereoselectively reducing the amine compound or enamine compound generated in the system.

The carbonyl compound used as a raw material for imine compound or enamine compound is, for example, those represented by general formula (8): 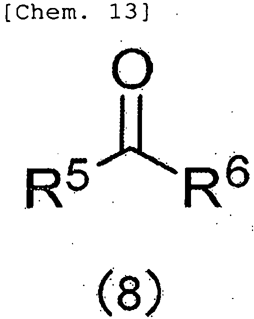 However, it is not necessarily limited thereto. Regarding general formula (8), R⁵ and R⁶ may be mutually identical or different, and denote a hydrogen atom, a hydrocarbyl group, an aryl group, or a heterocyclyl group, a carboxyl group, an ester group, an acyl group, in which one or more hydrogen atoms may be substituted by any substituents.

Specific examples of R⁵ and R⁶ include, but are not limited to, alkyl groups such as methyl, ethyl, propyl, butyl, pentyl, hexyl, heptyl, etc., fluoroalkyl groups such as trifluoroalkyl group, etc. , cycloalkyl groups such as cyclopropyl, cyclobutyl, cyclopentyl, cyclohexyl, etc., unsaturated hydrocarbons such as vinyl and allyl, aromatic mono- or polycyclic groups such as phenyl, 1-naphthyl, 2-naphthyl, anthryl, phenanthryl, indenyl, etc., hetero mono- or polycyclic groups such as thienyl, furyl, pyranyl, xanthenyl, pyridyl, pyrrolyl, imidazolinyl, indolyl, carbazoyl, phenanthrolinyl, etc., ferrocenyl group, carboxylic acid, ester groups such as methoxycarbonyl group, ethoxycarbonyl group, etc., acyl groups such as formyl group, acetyl group, benzoyl group, etc.

As substituents, any groups may be contained, including, but not limited to, hydrocarbon groups such as alkyl, alkenyl, cycloalkyl, cycloalkenyl, etc., fluoroalkyl group, halogen atom, oxygen-containing groups such as hydroxyl group, acyl group, alkoxy group, carboxyl group, ester group, etc., amino group, amide group, sulfonyl group, sulfenyl group, sulfo group, mercapto group, silyl group, nitro group, cyano group, etc. Furthermore, R⁵ and R⁶ may be bound to form a ring, and examples of such cases include, but are not limited to, saturated and unsaturated alicyclic groups that give cyclic ketones such as cyclopentanone, cyclohexanone, cycloheptanone, cyclopentanone, cyclohexenone, cycloheptenone, as well as saturated and unsaturated alicyclic groups that have a substituent having, at its respective carbon, an alkyl group, an aryl group, an unsaturated alkyl group, or a linear or cyclic hydrocarbon group comprising a heteroatom.

In order to achieve higher diastereoselectivity upon carrying out the reaction, they are preferably cyclic ketones having a substituent at their side chain, and such examples include cyclohexanone having a substituent at 2-position, cyclohexanone having a substituent at 3-position, cyclohexanone having a substituent at 4-position, cyclopentanone having a substituent at 2-position, cyclopentanone having a substituent at 3-position, azabicycloalkanone, adamantanone; more specifically, examples include 2-methyl cyclohexanone, 2-phenyl cyclohexanone, ethyl 2-cyclohexanone carboxylate, 3-methyl cyclohexanone, 4-phenyl cyclohexanone, 4-tert-butyl cyclohexanone, ethyl 4-cyclohexanone carboxylate, 1,4-cyclohexandion, ethyl cyclopentanone-2-carboxylate, 2-norbornanone, camphor, 8-azabicyclo[3.2.1]octane-3-on, tropinone, 3-quinuclidinone, 5-hydroxy-2-adamantanone, etc.
When a reductive amination reaction is carried out using the above cyclic ketones, cyclic amines are produced. Accordingly, the amine compound obtained from the reaction is preferably a cyclic amine or a cyclic diamine.

The amine compound as a raw material of the present invention is, for example, those represented by general formula (9) : 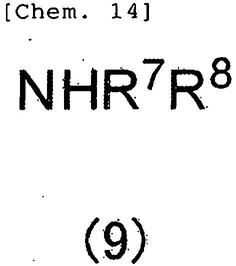 However, it is not limited thereto. Regarding general formula (9), R⁷ and R⁸ may be mutually identical or different, and they are a hydrogen atom, a hydrocarbyl group in which one or more hydrogen atoms may be substituted by any substituents, or a heterocyclyl group in which one or more hydrogen atoms may be substituted by any substituents.

Specific examples of R⁷ and R⁸ include, but are not limited to, alkyl groups such as methyl, ethyl, propyl, butyl, pentyl, hexyl, heptyl, etc., cycloalkyl groups such as cyclopropyl, cyclobutyl, cyclopentyl, cyclohexyl, etc., unsaturated hydrocarbons such as vinyl and allyl, aromatic mono- or polycyclic groups such as phenyl, 1-naphthyl, 2-naphthyl, anthryl, phenanthryl, indenyl, etc., hetero mono- or polycyclic groups such as thienyl, furyl, pyranyl, xanthenyl, pyridyl, pyrrolyl, imidazolinyl, indolyl, carbazoyl, phenanthrolinyl, etc., and ferrocenyl group, etc.

As substituents, any groups may be contained, including hydrocarbon groups such as alkyl, alkenyl, cycloalkyl, cycloalkenyl, etc., fluoroalkyl group, halogen atom, oxygen-containing groups such as hydroxyl group, acyl group, alkoxy group, carboxyl group, ester group, etc., and amino group, amide group, sulfonyl group, sulfenylgroup, sulfogroup, mercapto group, silyl group, nitro group, cyano group, etc. Furthermore, R⁷ and R⁸ may be bound to form a ring, and examples of such cases include, but are not limited to, saturated and unsaturated alicyclic groups that give cyclic ketones such as pyrrolidine, piperidine, azepane, pyrrole, tetrahydropyridine, tetrahydroazepine, etc., as well as saturated and unsaturated alicyclic groups that have a substituent having, at its respective carbon, an alkyl group, an aryl group, an unsaturated alkyl group, or a linear or cyclic hydrocarbon group comprising a heteroatom. In particular, in order to synthesize primary amines, in general, ammonium salt, benzylamine, racemic α-phenylethylamine, and optically-active α-phenylethylamine are used.

To carry out diastereoselective reaction, the carbonyl compound of general formula (8) and/or the amine compound of general formula (9) must have a chiral carbon; by appropriately combining these substrates, amine compounds can be diastereoselectively prepared.

The imine compound or enamine compound used in the invention may be easily obtained by, under the presence or absence of an acid catalyst, a condensation reaction of a carbonyl compound of general formula (8) with an amine compound of general formula (9).

As an acid catalyst, addition of Bronsted acid or Lewis acid is desirable. Preferable examples of Bronsted acid include organic acid such as carboxylic acid, sulfonic acid, or phenols, or mineral acid such as phosphoric acid, boric acid, hydrochloric acid or nitric acid, and specific examples include, but are not limited to, Bronsted acid such as formic acid, acetic acid, chloroacetic acid, trifluoroacetic acid, salicylic acid, p-toluenesulfonic acid, phenol and binaphthol, or Lexis acid such as titanium tetraisopropoxide and aluminium triisopropoxide. These may be used alone, or a combination of a plurality of these may be used. In particular, since formic acid reacts also as a hydrogen donator, it is a preferable Bronsted acid for reductive amination reaction of carbonyl compound and amine compound. Since acidic condition is preferred in the generation of imine compounds and enamine compounds, it is possible to use various buffers to make an acidic condition.

Hydrogen donators used in the present invention refer to a compound that can donate hydrogen by a thermal action or by a catalyst action, and the kind of such hydrogen-donating compound is not particularly limited. Examples of preferable hydrogen-donating compounds include, but are not limited to, methanol, ethanol, 1-propanol, 2-propanol, 1-butanol, sec-butyl alcohol, n-pentylalcohol, cyclopentylalcohol, n-hexylalcohol, cyclohexyl alcohol, benzyl alcohol, formic acid, HCOOK, HCOONa, HCOOLi and HCOONH₄. These may be used alone, or a combination of a plurality of these may be used. From the viewpoints of reactivity and economic efficiency, hydrogen-donating compounds are preferably formic acid or formate.

The amount of a hydrogen-donating compound used may be 1-30 equivalents relative to the carbonyl compound; from the viewpoints of reactivity and economic efficiency, it is preferably 1-10 equivalents.
When formate such as HCOOK, HCOONa, HCOOLi and HCOONH₄ is used as a hydrogen donator, a phase-transfer catalyst may be added to carry out the reaction if necessary. The phrase-transfer catalyst that can be used may be any salts having a long-chain alkyl ammonium cation; from the viewpoints of reactivity and economic efficiency, it is preferably a tetrabutylammonium salt.
In many cases, an effect of increased reaction rate is observed by the addition of a phase-transfer catalyst. The amount of the phase-transfer catalyst added is generally between 0.001-10 molar equivalents relative to the carbonyl compound; from the viewpoints of reactivity and economic efficiency, it is preferably 0.01-0.1 molar equivalents.

The amount of an amine compound used may be generally between 1-30 equivalents relative to the carbonyl compound; from the viewpoints of reactivity and economic efficiency, it is preferably 1-10 equivalents. When a gas such as ammonia is used as an amine compound, the gas may be used as is, or may be dissolved in an ammonia water or solvent, etc., or may be used as an ammonium salt.

Examples of ammonium salt used may be any salt containing ammonia, including ammonium formate, ammonium acetate, ammonium chloride, ammonium bromide, ammonium fluoride, ammonium iodide, ammonium phosphate, ammonium hexafluorophosphate, ammonium thiocyanate, ammonium benzoate, ammonium hydroxide, ammonium perchlorate, ammonium sulfate, ammonium nitrate, ammonium carbonate, ammonium hydrogen carbonate, etc.; from the viewpoints of reactivity and economic efficiency, it is preferably ammonium formate, ammonium acetate, or ammonium chloride.

A ruthenium, rhodium or iridium complex of general formula (1) used in the present invention may be prepared by mixing an organometallic compound of general formula (6): 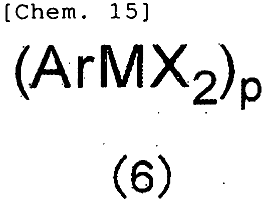 (wherein p denotes an integer of 2 or more) with a nitrogen-containing ligand of general formula (7):

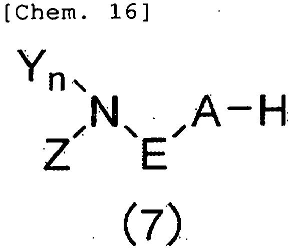

For example, a catalyst of general formula (1) of the present invention comprising the organometallic compound may be obtained as follows: under an inert gas atmosphere, an organometallic compound of general formula (6), a nitrogen-containing ligand of general formula (7), and a base are mixed in a halogen solvent, and stirred at room temperature; the resulting solution is washed with water, from which the solvent is distilled away, then dried under reduced pressure.

An amount of catalyst used can be expressed by a molar ratio S/C of a carbonyl compound relative to a ruthenium, rhodium or iridium catalyst (S denotes the number of moles of the carbonyl compound, imine compound or enamine compound, and C denotes the number of moles of the catalyst). In this case, the highest possible level of S/C varies significantly depending on the structure of a substrate, kind and concentration of a catalyst, reaction temperature, and kind of a hydrogen donator, etc.; practically, the S/C value should desirably be set at approximately 100-20000.

In the present invention, considering the physical and chemical properties of carbonyl compound, amine compound, imine compound, enamine compound, acid, hydrogen gas and hydrogen-donating compound, a reaction solvent may appropriately be used. Proticsolvent, non-proticsolvent, ionic liquid, water and buffer may be used alone, or a combination of a plurality of these may be used.

Regarding the reaction temperature, considering the solubility and reactivity of substrates and products as well as economic efficiency, reaction can be carried out preferably at approximately from -20°C to 100°C, and more preferably from 20°C to 80°C. Reaction time may vary depending on reaction conditions such as substrate concentration, temperature and pressure, etc.; reaction is completed within several minutes to 100 hours.

Purification of generated amine compounds may be performed using a publicly known method such as acid-base extraction, column chromatography, distillation, and re-crystallization, or an appropriate combination thereof. Under the presence of an organometallic compound of general formula (6) and an organic compound of general formula (7), it is possible to prepare an amine compound by reacting a carbonyl compound, an amine compound, with hydrogen gas or a hydrogen-donating organic or inorganic compound, or by reacting an imine compound or an enamine compound with hydrogen gas or a hydrogen-donating organic or inorganic compound.

### EXAMPLES

Hereinafter, examples are shown and the present invention is described in more detail; however, the present invention is not limited to these examples. Reactions described in each of the reference examples, comparative examples and examples below were performed under an atmosphere of inert gas such as argon gas or nitrogen gas. As a carbonyl compound and amine compound, commercially-available reagents were used as they were. Identification of ligand complexes and reaction products was performed using a nuclear magnetic resonance (NMR) apparatus, with tetramethylsilane (TMS) as the internal standard substance with its signal as δ=0 (δ denotes chemical shift). Conversion rates to amine compounds were determined by ¹H-NMR and gas chromatography (GC). Diastereoselectivity and steric isomers of an amine compound as a reaction product were determined by ¹H-NMR, GC, and using a Fourier transform infrared spectrometer (FT/IR). As a NMR apparatus, JNM-ECX-400P (JEOL Ltd.) was used; as a GC apparatus, GC-17A (Shimadzu Corporation) was used and as a Fourier transform infrared spectrometer, FT/IR-660 plus (JASCO Corporation) was used. Structures of the iridium catalyst, rhodium catalyst and ruthenium catalyst used in the preset invention are shown in Fig. 1.

### Reference Example 1: Synthesis of Cp*IrCl(N-(4-dimethylaminophenyl)-2-pyridylcarboxamide) complex (Ir-1)

400 mg (0.506 mmol)) of [Cp*IrCl₂]₂ (MW: 796.67) and 244 mg (1.01 mmol) of N-(4-dimethylaminoph-enyl)-2-pyridylcarboxamide (MW: 241.29) were introduced in a 20-mL Schlenk tube and subjected to argon-gas replacement. 10 mL of dehydrated methylene chloride and 140 µL (1.01 mmol) of triethylamine (MW: 101.19) were added and the mixture was stirred at room temperature for 20 h. After washing four times with a small amount of water, the organic solvent was distilled away, and the mixture was dried under reduced pressure. 20 ml of diisopropyl ether were added and the mixture was stirred at room temperature for 1 h, then the crystal was collected by filtration, washed with a small amount of diisopropyl ether, dried under reduced pressure to give 476 mg of orange powder crystal (78% isolated yield).
¹H NMR (400MHz, CDCl₃, δ/ppm): 1.42 (s. 15H), 2.92 (s, 6H), 6.74 (d, J=8.2Hz, 2H), 7.46 (ddd, J=7.3, 5.5, 1.8Hz, 1H), 7.50~7.58 (m, 2H), 7.89 (dt, J=7.3, 1.8Hz, 1H), 8:14 (d, J=7.8, 0.9Hz, 1H), 8.55 (d, J=5. 5Hz, 1H).
¹³C NMR (100MHz, CDCl₃, δ/ppm): 8.4, 41.2, 86.5, 112.8, 126.1, 127.0, 127.1, 138.4, 138.4, 147.9, 149.4, 155.8, 168.6.

### Reference Example 2: Synthesis of Cp*IrCl(N-(4-dimethylaminophenyl)-(S)-1-methyl-2-pyrrolidine carboxamide) complex (Ir-2)

250 mg (0.316 mmol) of [Cp*IrCl₂]₂ (MW: 796.67) and 313 mg (1.27 mmol) of N-(4-dimethylaminophenyl)-(S)-1-methyl-2-pyrrolidinecarboxam ide (MW: 247.34) were introduced in a 20-mL Schlenk tube and subjected to argon-gas replacement. 5 mL of dehydrated methylene chloride and 88 µL (0.6.32 mmol) of triethylamine (MW: 101.19.) were added and the mixture was stirred at room temperature for 24 h. After washing four times with a small amount of water, the organic solvent was distilled away, and the mixture was dried under reduced pressure. 20 ml of diisopropyl ether were added and the mixture was stirred at room temperature for 1 h, then the crystal was collected by filtration, washed with a small amount of diisopropyl ether, dried under reduced pressure to give 189 mg of orange powder crystal (50% isolated yield).
¹H NMR (400MHz, CDCl₃, δ/ppm): 1.34 (s, 15H), 1.75~2.15 (m, 3H), 2.45~2.60 (m, 1H), 2.89 (s, 6H), 3.03 (s, 3H), 3.25∼3.45 (m, 2H), 4.06 (d, J=8.7Hz, 1H), 6.60∼6.80 (brs, 2H), 6.80∼7.00 (brs, 1H), 7.12∼7.35 (brs, 1H).
¹³C NMR (100MHz, CDCl₃, δ/ppm): 8.6, 22.5, 23.8, 41.3, 47.3, 63.9, 72.8, 85.3, 112.4, 113.7, 127.3, 128.1, 140.1, 148.0, 178.8.

### Reference Example 3 : Synthesis of Cp*RhCl(N-(4-dimethylaminophenyl)-2-pyridylcarboxamide) complex (Rh-1)

150 mg (0.243 mmol) of [Cp*RhCl₂]₂ (MW: 618.08) and 117 mg (0.485 mmol) of N-(4-dimethylaminophenyl)-2-pyridylcarboxamide (MW: 241.29) were introduced in a 20-mL Schlenk tube and subjected to argon-gas replacement. 5 mL of dehydrated methylene chloride and 68 µL (0.485 mmol) of triethylamine (MW: 101.19) were added and the mixture was stirred at room temperature for 20 h. After washing four times with a small amount of water, the organic solvent was distilled away, and the mixture was dried under reduced pressure. 20 ml of diisopropyl ether were added and the mixture was stirred at room temperature for 1 h, then the crystal was collected by filtration, washed with a small amount of diisopropyl ether, dried under reduced pressure to give 211 mg of orange powder crystal (85% isolated yield).
¹H NMR (400MHz, CDCl₃, δ/ppm): 1.42 (s, 15H), 2.92 (s, 6H), 6.72∼6.81 (m, 2H), 7.49 (ddd, J=7.3, 5.5, 1.8Hz, 1H), 7.60∼7.68 (m, 2H), 7.91 (dt, J=7.8, 1.8Hz, 1H), 8.13 (dd, J=7.8, 0.9Hz, 1H), 8.61 (d, J=5.5Hz, 1H).
¹³CNMR (100MHz, CDCl₃/ δ/ppm) : 8.6, 41.3, 94.5, 94.5, 112.8, 125.7, 126.6, 127.5, 138.5, 138.6, 147.7, 149.5, 156.8, 166.6.

### Reference Example 4 Synthesis of RuCl(N-(4-dimethylaminophenyl)-2-pyridylcarboxamide)(p-cymen e) complex (Ru-1)

200 mg (0.327 mmol) of [RuCl₂(p-cymene)]₂ (MW: 612.39) and 140 mg (0.653 mmol) of N-(4-dimethylaminophenyl)-2-pyridylcarboxamide (MW: 241.29) were introduced in a 20-mL Schlenk tube and subjected to argon-gas replacement. 5 mL of dehydrated methylene chloride and 91 µL (0.653 mmol) of triethylamine (MW: 101.19) were added and the mixture was stirred at room temperature for 20 h. After washing four times with a small amount of water, the organic solvent was distilled away, and the mixture was dried under reduced pressure. 20 ml of the mixed solvent of 2-propanol:dichloromethane = 20:1 were added and stirred at room temperature for 1 h, then the crystal was collected by filtration, dried under reduced pressure to give 199 mg of orange powder crystal (60% isolated yield).
¹H NMR (400MHz, CDCl₃, δ/ppm) : 1.05 (d, J=6.9Hz, 3H), 1.06 (d, J=6.9Hz, 3H), 2.20 (s, 3H), 2.58 (quintet, J=6.9Hz, 1H), 2.96 (s, 6H), 4.69 (d, J=6.0Hz, 1H), 5.14 (d, J=6.0Hz, 1H), 5.17 (d, J=6.0Hz, 1H), 5.22 (d, J=6.0Hz, 1H), 6.73∼6.84 (m, 2H), 7.42 (ddd, J=7.3, 5.5, 1.8Hz, 1H), 7.50∼7.58 (m, 2H), 7.88 (dt, J=7.8, 1.4Hz, 1H)8.07 (dd, J=7.8, 0.9Hz, 1H) 8.94 (d, J=5.5Hz, 1H). ¹³C NMR (100MHz, CDCl₃, δ/ppm) : 18.8, 21.8, 22.3, 30.7, 41.1, 83.4, 84.1, 85.0, 100.2, 101.2, 112.9, 125.6, 126.2, 126.6, 138.2, 142.7, 147.7, 153.0, 156.1, 167.0.

### Reference Example 5 :Synthesis of Cp*IrCl(N-(4-dimethylaminophenyl)-(S)-2-pyrrolidinecarboxami de) complex (Ir-7)

200 mg (0.253 mmol) of [Cp*IrCl₂]₂ (MW: 796.67) and 130 mg (0.556 mmol) of N-(4-dimethylaminophenyl)-(S)-2-pyrrolidinecarboxamide (MW: 233.31) were introduced in a 20-mL Schlenk tube and subjected to argon-gas replacement. 5 mL of dehydrated methylene chloride and 71 µL (0.506 mmol) of triethylamine (MW: 101.19) were added and the mixture was stirred at room temperature for 22 h. After washing four times with a small amount of water, the organic solvent was distilled away, and the mixture was dried under reduced pressure. 20 ml of diisopropylether were added and stirred at room temperature for 1 h, then the crystal was collected by filtration, washed with a small amount of diisopropylether, dried under reduced pressure to give 236 mg of orange powder crystal (71% isolated yield).
¹H NMR (400MHz, CDCl₃, δ/ppm) : 1.38 (s, 15H), 1.65∼1.85 (m, 1H), 1.85~2.05 (m, 1H), 2.05~2.20 (m, 1H), 2.20~2.35 (m, 1H), 2.89 (s, 6H), 3.00∼3.20 (brs, 1H), 3.40∼3.55 (brs, 1H), 4.12 (quartet, J=8.2Hz, 1H), 4.75~5.25 (brs, 1H), 6.69 (d, J=9.2Hz, 2H), 7.17 (d, J=9.2Hz, 2H).
¹³C NMR (100MHz, CDCl₃, δ/ppm) : 8.7, 26.6, 29.6, 41.4, 54.6, 64.5, 85.2, 112.8, 128.0, 139.7, 147.8, 179.8.

### Comparative Example 1: Synthesis of 4-tert-butylcyclohexylamine

771 mg (5.0 mmol) of 4-tert-butylcyclohexanone (MW: 154.25) and 154 mg of 10% Pd/C (N. E. Chemcat Corporation, NX type, 50wt% hydrous product) were introduced in a 100-mL pressure-resistant reactor and subjected to argon-gas replacement. 7 mL of a 7N ammonia-methanol solution were added, and stirred under hydrogen pressure of 1.0 MPa and reaction temperature of 30°C for 15 h. The reaction solution was filtered through Celite to remove the 10% Pd/C, and methanol was added to make exactly the 100-mL methanol solution. A 10-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 24% yield and a diastereoselectivity of cis:trans = 31:69.

### Comparative Example 2 Synthesis of 4-tert-butylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid, and 29.8 mg (0.050 mmol, S/C=100) of iridium catalyst Ir-7 (MW: 595.20) were added and stirred while heating at 60°C for 5 h. The solvent was distilled away from the reaction solution, 7.5 mL of 1M KOH aqueous solution were added, and the resulting solution was extracted with 40 mL of dichloromethane, the organic layer was washed twice with 5 mL of water, then dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard; however, no 4-tert-butylcyclohexylamine was generated although the conversion rate of the raw material was 92%.

### Example 1: Synthesis of 2-methylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 603 µL (5.0 mmol) of 2-methylcyclohexanone (MW: 112.17), 286 µL (5.0 mmol) of acetic acid (MW: 60.05), and 6.03 mg (0.010 mmol, S/C=500) of iridium catalyst Ir-1 (MW: 603.18) were added and stirred while heating at 60°C for 4 h. After the reaction solution was processed with KOH aqueous solution, GC analysis was performed, and 2-methylcyclohexylamine was obtained with 82% yield and a diastereoselectivity of cis:trans = 94:6.

### Example 2: Synthesis of 2-phenylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 871 mg (5.0 mmol) of 2-phenylcyclohexanone (MW: 174.24), 286 µL (5.0 mmol) of acetic acid (MW: 60.05), and 6.03 mg (0.010 mmol, S/C=500) of iridium catalyst Ir-1 (MW: 603.18) were added and stirred while heating at 60°C for 17 h. The solvent was distilled away from the reaction solution, 7.5 mL of 1M KOH aqueous solution were added, and the resulting solution was extracted with 40 mL of dichloromethane, the organic layer was washed twice with 5 mL of water, then dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, confirming that 2-phenylcyclohexylamine was obtained with 81% yield and a diastereoselectivity of cis:trans = 98:2.

### Example 3: Synthesis of 2-chlorocyclohexylamine

0.946g (15.0 mmol) of ammonium formate (MW: 63.06) were introduced in a 20-mL Schlenk tube, dried under reduced pressure, then subjected to argon-gas replacement. 5 mL of methanol, 571 µL (5.0 mmol) of 2-chlorocyclohexanone (MW: 132.59), 286 µL (5.0 mmol) of acetic acid (MW: 60.05), and 6.03 mg (0.010 mmol, S/C=500) of iridium catalyst Ir-1 (MW: 603.18) were added and stirred while heating at 40°C for 18 h. The solvent was distilled away from the reaction solution, 7.5 mL of 1M KOH aqueous solution were added, and the resulting solution was extracted with 40 mL of dichloromethane, the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 2-chlorocyclohexylamine with 61% yield. The obtained objective substance was induced in hydrochloride, and IR measurement was carried out, confirming that the major product was a cis form.

### Example 4: Synthesis of ethyl 2-aminocyclohexane carboxylate

0.946 g (15.0 mmol) of ammonium formate (MW: 63.06) were introduced in a 20-mL Schlenk tube, dried under reduced pressure, then subjected to argon-gas replacement. 5 mL of methanol, 795 µL (5.0 mmol) of ethyl 2-cyclohexanone carboxylate (MW: 170.21), 286 µL (5.0 mmol) of acetic acid (MW: 60.05), and 6.03 mg (0.010 mmol, S/C=500) of iridium catalyst Ir-1 (MW: 603.18) were added and stirred while heating at 60°C for 8 h. The solvent was distilled away from the reaction solution, 7.5 mL of saturated NaHCO₃ aqueous solution were added, and the resulting solution was extracted with 50 mL of dichloromethane. The organic layer was washed twice with 5 mL of water, dried with sodium sulfate, and after the drying agent was removed by filtration, dichloromethane was added to make exactly the 50-mL dichloromethane solution. A 5-mL aliquot of this solution was collected, from which the solvent was distilled away, and the resulting solution was subjected to ¹H NMR quantification using coumarin (MW : 146.14) as the internal standard, confirming that ethyl 2-aminocyclohexane carboxylate was obtained with 54% yield and a diastereoselectivity of cis:trans = 96:4.

### Example 5: Synthesis of 3-methylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 610 µL (5.0 mmol) of 2-mechylcyclohexanone (MW: 112.17), 286 µL (5.0 mmol) of acetic acid (MW: 60.05) and 6.03 mg (0.010 mmol, S/C=500) of iridium catalyst Ir-1 (MW: 603.18) were added and stirred while heating at 60°C for 4 h. The solvent was distilled away from the reaction solution, 20 mL of 1M KOH solution were added, and the resulting solution was extracted with 40 mL of dichloromethane; the organic layer was washed twice with 5 mL of water, dried with sodium sulfate, filtered, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 3-methylcyclohexylamine with 75% yield and a diastereoselectivity of cis:trans = 4:96.

### Example 6 : Synthesis of 4-tert-butylcyclohexylamine

0.946g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05) and 6.03 mg (0.010 mmol, S/C=500) of iridium catalyst Ir-1 (MW: 603.18) were added and stirred while heating at 60°C for 4 h. The solvent was distilled away from the reaction solution, 7. 5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 97% yield and 99% or more of cis form. Its diastereoselectivity is superior to that of Comparative example 1, showing effectiveness of the present invention.

### Example 7: Synthesis of 4-tert-butylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 12.7 mg (0.025 mmol, S/C=200) of iridium catalyst Ir-3 (MW: 507.05) were added and stirred while heating at 60°C for 5 h. The solvent was distilled away from the reaction solution, 7. 5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 82% yield and a diastereoselectivity of cis:trans = 88:12. This diastereoselectivity is significantly superior to that of Comparative example 1, showing effectiveness of the present invention.

### Example 8 : Synthesis of 4-tert-butylcyclohexylamine

0.946g (15.0mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 24.2 mg (0.05 mmol, S/C=100) of iridium catalyst Ir-5 (MW: 485.00) were added and stirred while heating at 60°C for 4 h. The solvent was distilled away from the reaction solution, 7. 5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 92% yield and a diastereoselectivity of cis:trans = 95:5. This diastereoselectivity is significantly superior to that of Comparative example 1, showing effectiveness of the present invention.

### Example 9 : Synthesis of 4-tert-butylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 26.7 mg (0.05 mmol, S/C=100) of iridium catalyst Ir-6 (MW: 534.07) were added and stirred while heating at 60°C for 4 h. The solvent was distilled away from the reaction solution, 7.5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 80% yield and a diastereoselectivity of cis:trans = 93:7. This diastereoselectivity is significantly superior to that of Comparative example 1, showing effectiveness of the present invention.

### Example 10 : Synthesis of 4-tert-butylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 30.5 mg (0.050 mmol, S/C=100) of iridium catalyst Ir-2 (MW: 609.22) were added and stirred while heating at 60°C for 5 h. The solvent was distilled away from the reaction solution, 7.5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 93% yield and 99% or more of cis form. Its diastereoselectivity and reactivity are superior to those of Comparative examples 1 and 2, showing effectiveness of the present invention.

### Example 11: Synthesis of 4-tert-butylcyclohexylamine

0.946g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 5.14 mg (0.010 mmol, S/C=500) of rhodium catalyst Rh-1 (MW: 513.87) were added and stirred while heating at 60°C for 5 h. The solvent was distilled away from the reaction solution, 7. 5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 92% yield and a diastereoselectivity of cis:trans = 98:2. This diastereoselectivity is significantly superior to that of Comparative example 1, showing effectiveness of the present invention.

### Example 12 : Synthesis of 4-tert-butylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 771 mg (5.0 mmol) of 4-tert-butylcyclohexanone (MW: 154.25), 286 µL (5.0 mmol) of acetic acid (MW: 60.05), and 25.5 mg (0.050 mmol, S/C=100) of ruthenium catalyst Ru-1 (MW: 511.02) were added and stirred while heating at 60°C for 7 h. The solvent was distilled away from the reaction solution, 7. 5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-tert-butylcyclohexylamine with 74% yield and a diastereoselectivity of cis:trans = 95:5. This diastereoselectivity is significantly superior to that of Comparative example 1, showing effectiveness of the present invention.

### Example 13: Synthesis of 4-phenylcyclohexylamine

0.946 g (15.0 mmol) of ammonium formate (MW: 63.06) were introduced in a 20-mL Schlenk tube, dried under reduced pressure, then subjected to argon-gas replacement. 5 mL of methanol, 871 mg (5.0 mmol) of 4-phenylcyclohexanone (MW: 174.24), 286 µL (5.0 mmol) of acetic acid (MW: 60.05), and 6.03 mg (0.010 mmol, S/C=500) of iridium catalyst Ir-1 (MW: 603.18) were added and stirred while heating at 60°C for 4 h. The solvent was distilled away from the reaction solution, 7.5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 4-phenylcyclohexylamine with 99% yield and a diastereoselectivity of cis:trans = 95:5.

### Example 14: Synthesis of 4-methylcyclohexylamine

0.946g (15.0 mmol) of ammonium formate (MW: 63.06) were introduced in a 20-mL Schlenk tube, dried under reduced pressure, then subjected to argon-gas replacement. 5 mL of methanol, 610 µL (5.0 mmol) of 4-methylcyclohexanone (MW: 112.17), 286 µL (5.0 mmol) of acetic acid (MW: 60.05), and 6.03 mg (0.010 mmol, S/C=500) of iridium catalyst Ir-1 (MW: 603.18) were added and stirred while heating at 60°C for 4 h. After processing the reaction solution with a KOH aqueous solution, GC analysis was performed, giving 4-methylcyclohexylamine with 75% yield and a diastereoselectivity of cis:trans = 90:10.

### Example 15: Synthesis of 2-amino norbornane

0.946 g (15.0 mmol) of ammonium formate (MW: 63.06) where introduced in a 20-mL Schlenk tube, dried under reduced pressure, then subjected to argon-gas replacement. 5 mL of methanol, 551 mg (5.0 mmol) of 2-norbornanone (MW: 110.15), 286 µL (5.0 mmol) of acetic acid (MW: 60.05), and 6.03 mg (0.010 mmol, S/C=500) of iridium catalyst Ir-1 (MW: 603.18) were added and stirred while heating at 40°C for 17 h. The solvent was distilled away from the reaction solution, 7.5 mL of 1M KOH aqueous solution were added, and the resulting solution was extracted with 40 mL of dichloromethane; the organic layer was washed twice with 5 mL of water, and dichloromethane was added to make exactly the 50-mL dichloromethane solution. A 5-mL aliquot of this solution was collected, from which the solvent was distilled away, and the resulting solution was subjected to ¹H NMR quantification using coumarin (MW: 146.14) as the internal standard, giving 2-amino norbornane with 62% yield and a diastereoselectivity of exo:endo = 94:6.

### Example 16: Synthesis of 1,4-cyclohexanediamine

1.892 g (30.0 mmol) of ammonium formate (MW: 63.06) were introduced in a 20-mL Schlenk tube, dried under reduced pressure, then subjected to argon-gas replacement. 5 mL of methanol, 561 mg (5.0 mmol) of 1,4-cyclohexanedione (MW: 112.13), 573 µL (10.0 mmol) of acetic acid (MW: 60.05), and 6.03 mg (0.010 mmol, S/C=500) of iridium catalyst Ir-1 (MW: 603.18) were added and stirred while heating at 60°C for 4 h. Methanol was added to make exactly the 50-mL methanol solution; a 5-mL aliquot of this solution was collected, from which the solvent was distilled away, and the resulting solution was subjected to ¹H NMR quantification using coumarin (MW: 146.14) as the internal standard, giving 1, 4-cyclohexanediamine with 49% yield and 99% or more of cis form.

### Example 17: Synthesis of 1,4-cyclohexane dibenzylamine

1145 µL (2.1 mmol) of benzylamine (MW: 107.15) were introduced in a 20-mL Schlenk tube, dried under reduced pressure, then subjected to argon-gas replacement. 5 mL of methanol, 561 mg (5.0 mmol) of 1,4-cyclohexanedione (MW: 112.13), 755 µL (20.0 mmol) of formic acid (MW: 46.03), and 6.03 mg (0.010 mmol, S/C=500) of iridium catalyst Ir-1 (MW: 603.18) were added and stirred while heating at 60°C for 21 h. The solvent was distilled away from the reaction solution, 25 mL of 1M KOH aqueous solution were added, and the resulting solution was extracted with 80 mL of dichloromethane; the organic layer was washed twice with 10 mL of water, and dichloromethane was added to make exactly the 100-mL dichloromethane solution. A 10-mL aliquot of this solution was collected, from which the solvent was distilled away, and the resulting solution was subjected to ¹H NMR quantification using coumarin (MW: 146.14) as the internal standard, giving 1,4-cyclohexane dibenzylamine with 99% yield and a diastereoselectivity of cis:trans = 97:3.

### Example 18: (2R)-3-methyl-N-((R)-1-phenylethyl)butane-2-amine

265 µL (2.1 mmol) of (R)-1-phenethylamine (MW: 121.18) was introduced in a 20-mL Schlenk tube, dried under reduced pressure, then subjected to argon-gas replacement. 4 mL of dehydrated ethyl acetate, 214 µL (2.0 mmol) of 3-methyl-2-butanone (MW: 86.13), 226 µtL (6.0 mmol) of formic acid (MW: 46.03), and 12.3 mg (0.020 mmol, S/C=100) of iridium catalyst Ir-1 (MW: 603.18) were added and stirred while heating at 60°C for 21 h. The solvent was distilled away from the reaction solution, 10 mL of 1M KOH aqueous solution were added, and the resulting solution was extracted with 15 mL of dichloromethane; the organic layer was washed twice with 3 mL of water, and dichloromethane was added to make exactly the 20-mL dichloromethane solution. A 5-mL aliquot of this solution was collected, from which the solvent was distilled away, and the resulting solution was subjected to ¹H NMR quantification using coumarin (MW: 146.14) as the internal standard, giving 3-methyl-N-((R)-1-phenylethyl)butane-2-amine with 80% yield and a diastereoselectivity of (R,R) : (R,S) = 87:13.

### Example 19: (2R)-3-methyl-N-((R)-1-phenylethyl)butane-2-amine

290 µL (2.3 mmol) of (R)-1-phenethylamine (MW: 121.18) were introduced in a 20-mL Schlenk tube, dried under reduced pressure, then subjected to argon-gas replacement. 4 mL of dehydrated ethyl acetate, 214 µL (2.0 mmol) of -3-methyl-2-butanone (MW: 86.13), 226 µL (6.0 mmol) of formic acid (MW: 46.03) and 2.0 mg (0.004 mmol, S/C=500) of iridium catalyst Ir-3 (MW: 507.05) were added and stirred while heating at 60°C for 21 h. 10 mL of 1M KOH aqueous solution were added, and the resulting solution was extracted with 15 mL of dichloromethane; the organic layer was washed twice with 3 mL of water, and dichloromethane was added to make exactly the 20-mL dichloromethane solution. A 5-mL aliquot of this solution was collected, from which the solvent was distilled away, and the resulting solution was subjected to ¹H NMR quantification using coumarin (MW: 146.14) as the internal standard, giving 3-methyl-N-((R)-1-phenylethyl)butane-2-amine with 96% yield and a diastereoselectivity of (R, R) : (R, S) = 80:20.

### Example 20: Synthesis of 4-(1-phenyl-ethylamino)-adamantane-1-ol

1.662g (10.0 mmol) of 5-hydroxyadamantane-2-one (MW: 166.22) were introduced in a 50-mL Schlenk tube, dried under reduced pressure, then subjected to argon-gas replacement. 20 mL of dehydrated ethyl acetate, 1 .45 mL (11. 5 mmol) of DL-α-methylbenzylamine (MW: 121.18, d 0.96), 755 µL (20.0 mmol) of formic acid (MW: 46.03, d 1.220), and 2.54 mg (0.005 mmol, S/C=2000) of iridium catalyst Ir-3 (MW: 507.05) were added and stirred while heating at 50°C for 24 h. The solvent was distilled away from the reaction solution, 15 mL of 1M KOH aqueous solution were added, and the resulting solution was extracted with 50 mL of dichloromethane, washed once with 10 mL of water, and methylene chloride was added to make exactly the 100-mL methylene chloride solution. A 10-mL aliquot of this solution was collected, from which the solvent was distilled away, and the resulting solution was subjected to ¹H NMR quantification using coumarin (MW: 146.14) as the internal standard, giving 4-(1-phenyl-ethylamino)-adamantane-1-ol with 99% yield and a diastereoselectivity of cis:trans = 75:25.

### Example 21: Synthesis of 4-(1-phenyl-ethylamino)-adamantane-1-ol

831 mg (5.0 mmol) of 5-hydroxyadamantane-2-one (MW: 166.22) and 25.4 mg (0.05 mmol, S/C=100) of iridium catalyst Ir-3 (MW: 507.05) were introduced in a 100-mL pressure-resistant reactor and subjected to argon-gas replacement. A solution consisting of 631 µL (5.0 mmol) of DL-α-methylbenzylamine (MW: 121.18, d 0.96), 473 mg (5.0 mmol) of chloroacetic acid (MW: 94.50) and 6 mL of dehydrated methanol was added, and stirred under hydrogen pressure of 1.0 MPa and reaction temperature of 50°C for 18 h. The solvent was distilled away from the reaction solution, 8 mL of 1M KOH aqueous solution were added, and the resulting solution was extracted with 50 mL of dichloromethane; the organic layer was washed once with 10 mL of water, and methylene chloride was added to make exactly the 100-mL methylene chloride solution. A 10-mL aliquot of this solution was collected, from which the solvent was distilled away, and the resulting solution was subjected to ¹H NMR quantification using coumarin (MW: 146.14) as the internal standard, giving 4-(1-phenyl-ethylamino)-adamantane-1-ol with 99% yield and a diastereoselectivity of cis:trans = 71:29.

### Example 22: Synthesis of 4-(1-phenyl-ethylamino)-adamantane-1-ol

831 mg (5.0 mmol) of 5-hydroxyadamantane-2-one (MW: 166.22) and 24. 2 mg (0.05 mmol, S/C=100) of iridium catalyst Ir-4 (MW: 483.99) were introduced in a 100-mL pressure-resistant reactor and subjected to argon-gas replacement. A solution consisting of 726 µL (5.75 mmol) of DL-α-methylbenzylamine (MW: 121.18, d0.96), 543 mg (5.75 mmol) of chloroacetic acid (MW: 94.50) and 6 mL of dehydrated methanol was added, and stirred under hydrogen pressure of 1.0 MPa and reaction temperature of 50°C for 18 h. The solvent was distilled away from the reaction solution, 8 mL of 1M KOH aqueous solution were added, and the resulting solution was extracted with 50 mL of dichloromethane; the organic layer was washed once with 10 mL of water, and methylene chloride was added to make exactly the 100-mL methylene chloride solution. A 10-mL aliquot of this solution was collected, from which the solvent was distilled away, and the resulting solution was subjected to ¹H NMR quantification using coumarin (MW: 146.14) as the internal standard, giving 4-(1-phenyl-ethylamino)-adamantane-1-ol with 97% yield and a diastereoselectivity of cis:trans = 68:32.

As described above in detail, this invention enables to produce an amine compound efficiently and highly diastereoselectively by reductive amination reaction of carbonyl compound and amine compound.

## Claims

1. A process for preparing an amine compound, **characterized in that** an imine compound or an enamine compound is diastereoselectively reduced using hydrogen gas or an organic or inorganic hydrogen-donating compound, under the presence of an organometallic compound of general formula (1): 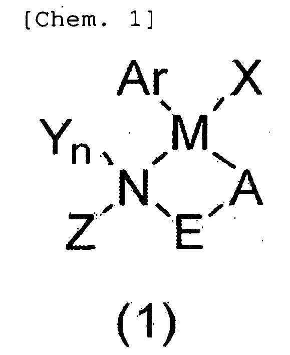 wherein in general formula (1), Ar is a cyclopentadienyl group or an aromatic compound, in which one or more hydrogen atoms may be substituted by a substituent W, and W denotes a saturated or unsaturated C1-10 hydrocarbon group, an aryl group, a heterocyclyl group, an alkoxy group, a fluoroalkyl group, an acyl group, an ester group, a hydroxyl group, an amino group, an amide group, a carboxyl group, a sulfonyl group, a nitro group, a cyano group, a sulfenyl group, a sulfo group, a mercapto group, a phosphino group, a silyl group or a halogen group, M is ruthenium, rhodium, or iridium, X is a hydride group or an anionic group, E denotes a link group which is a saturated or unsaturated C1-6 hydrocarbon group in which one or more hydrogen atoms may be substituted by a substituent W, or which is an arylene group in which one or more hydrogen atoms may be substituted by C1-10 alkyl group, cycloalkyl group, aryl group, alkenyl group, acyl group, halogen group, ester group, amino group, amide group, carboxyl group, hydroxyl group, nitro group, cyano group, sulfenyl group, sulfo group, or thiol group, wherein one or more hydrogen atoms in each of said substituents may further be substituted by W, A is an amide group having a binding mode of "M-NR¹C (O) -E, " or an oxygen atom, wherein when A is an oxygen atom, E is an arylene group in which one or more hydrogen atoms may be substituted by a substituent W, R¹ denotes a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W, wherein one or more hydrogen atoms in the substituent W may further be substituted by a substituent W, Y and Z are identical or mutually different, and denote a C1-10 alkyl group, a cycloalkyl group, an aryl group, a heterocyclyl group, an alkenyl group, or a silyl group, in which one or more hydrogen atoms may be substituted by a substituent W, wherein "Y and Z," "Z and E," "Y and E," or "Y-Z-E" may be bound to form a ring, and n denotes an integer of 0 or 1, wherein when n=0, the bond between N-Z or N-E is a double bond.

2. The process for preparing an amine compound according to Claim 1, **characterized in that** a part of formula (1) expressed by general formula (2) below: 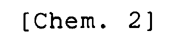 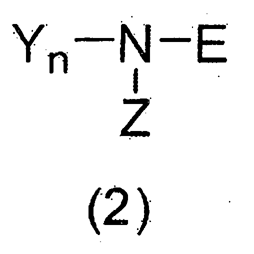 wherein in general formula (2), n=0 and N, E and Z have the same meanings as defined in claim 1, is a group comprising a nitrogen-containing cyclic group.

3. The process for preparing an amine compound according to Claim 1 or 2, **characterized in that** the organometallic compound of formula (1) is a compound of general formula (3): 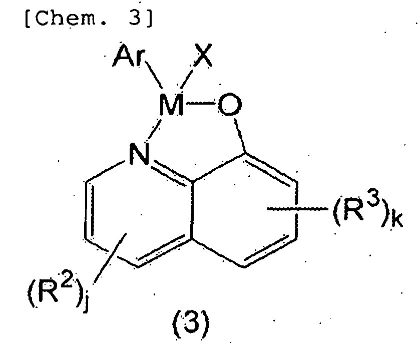 wherein in general formula (3), Ar, M and X have the same meanings as defined in Claim 1, R² and R³ are mutually identical or mutually different, and denote a hydrogen atom, a halogen atom, a nitro group, a cyano group, a hydroxyl group, a sulfo group, a mercapto group, a carboxyl group, or the following groups in which one or more hydrogen atoms may be substituted by a substituent W: a C1-10 alkyl group, a cycloalkyl group, an aryl group, a heterocyclyl group, an alkenyl group, an alkynyl group, an alkoxy group, an ester group, a fluoroalkyl group, an acyl group, a sulfonyl group, an amino group, an amide group, a phosphino group, a sulfenyl group, or a silyl group, W has the same meaning as described in Claim 1, j and k are, independently of each other, an integer from 0 to 3, wherein one or more carbon atoms, which is not bonded to R² or R³, in the quinoline ring may be replaced by a nitrogen atom, and when j and/or k is(are) 1 or more, R² and R³ may be linked each other to form a ring, and when j and/or k is (are) 2 or more, two or more of R² and/or R³ may be mutually linked to form a ring,
or one or more organometallic compounds selected from the group consisting of the organometallic compound of general formula (4) below: 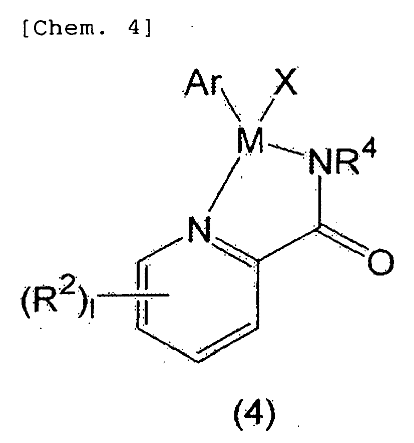 wherein in general formula (4), Ar, M and X have the same meanings as defined in Claim 1, R² has the same meaning as the above R² and R³, R⁴ denotes a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W, W has the same meaning as described in Claim 1, 1 denotes an integer from 0 to 4, wherein one or more carbon atoms, which is not bonded to R², in the pyridine ring may be replaced by a nitrogen atom, and wherein when 1 is 2 or more, two or more of R² may be linked each other to form a ring,
and general formula (5) below: 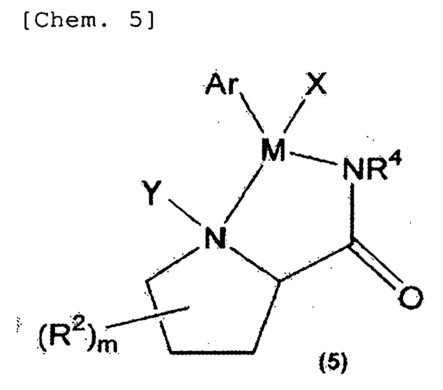 wherein in general formula (5), Ar, M, X and Y have the same meanings as defined in Claim 1, R² has the same meaning as the above R² and R³, R⁴ denotes a hydrogen atom, a C1-10 alkyl group, a cycloalkyl group, an aryl group, a heterocyclyl group, an alkenyl group, an alkynyl group, an ester group, an acyl group, an amide group, a phosphino group, a sulfenyl group, a sulfinyl group, a sulfonyl group or a silyl group, in which one or more hydrogen atoms may be substituted by a substituent W, W has the same meaning as described in Claim 1, m denotes an integer from 0 to 7, wherein one or more carbon atoms in the pyrrolidine ring may be replaced by a nitrogen atom, and wherein when m is 2 or more, two or more of R² may be linked each other to form a ring.

4. A process for preparing an amine compound, **characterized in that** an imine compound or an enamine compound is diastereoselectively reduced using hydrogen gas or an organic or inorganic hydrogen-donating compound, under the presence of an organometallic compound of general formula (6) : 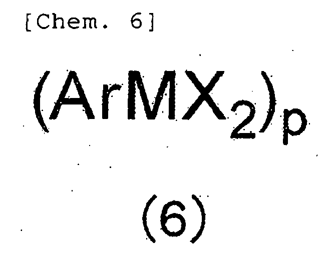 wherein in general formula (6), Ar, M and X have the same meanings as defined in Claim 1, and p denotes an integer of 2 or more, and an organic compound of general formula (7): 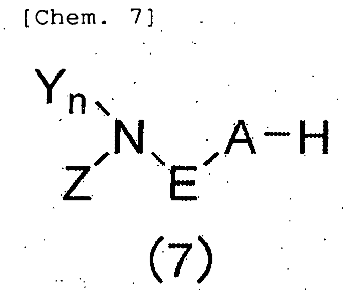 wherein in general formula (7), A, E, Y, Z and n have the same meanings as defined in Claim 1.

5. The process for preparing an amine compound or according to any one of Claims 1 to 4, **characterized in that** a carbonyl compound is mixed with an amine compound or an ammonium salt, and the imine compound or enamine compound generated in this system is diastereoselectively reduced.

6. The process for preparing an amine compound according to any one of Claims 1 to 5, **characterized in that** M in formula (1) and formula (6) is rhodium or iridium.

7. The process for preparing an amine compound according to any one of Claims 1 to 6, **characterized in that** the organic or inorganic hydrogen-donating compound is formic acid or formate.

8. The process for preparing an amine compound according to any one of Claims 1 to 7, **characterized in that** the amine compound obtained from the reaction is a cyclic amine compound or a cyclic diamine compound.

## Patentansprüche

1. Verfahren zur Herstellung einer Aminoverbindung, **dadurch gekennzeichnet, dass** eine Iminverbindung oder Enaminverbindung diastereoselektiv mit Wasserstoffgas oder einer organischen oder anorganischen Wasserstoff abgebenden Verbindung unter Vorhandensein einer metallorganischen Verbindung der folgenden allgemeinen Formel (1)reduziert wird: 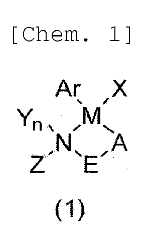 worin in der allgemeinen Formel (1) Ar eine Cyclopentadienylgruppe oder eine aromatische Verbindung ist, in der ein oder mehrere Wasserstoffatome durch einen Substituenten W substituiert werden können und W eine gesättigte oder ungesättigte CL-10-Kohlenwasserstoffgruppe, eine Arylgruppe, eine Heterocyclylgruppe, eine Alkoxygruppe, eine Fluoroalkylgruppe, eine Acylgruppe, eine Estergruppe, eine Hydroxylgruppe, eine Aminogruppe, eine Amidgruppe, eine Carboxylgruppe, eine Sulfonylgruppe, eine Nitrogruppe, eine Cyangruppe, eine Sulfenylgruppe, eine Sulfogruppe, eine Mercaptogruppe, eine Phosphingruppe, eine Silylgruppe oder eine Halogengruppe bezeichnet, M Ruthenium, Rhodium oder Iridium ist, X eine Hydridgruppe oder eine anionische Gruppe ist, E eine Verbindungsgruppe bezeichnet, die eine gesättigte oder ungesättigte C1-6-Kohlenwasserstoffgruppe ist, in der ein oder mehrere Wasserstoffatome durch einen Substituenten W substituiert werden können, oder eine Arylengruppe ist, in der ein oder mehrere Wasserstoffatome durch eine C1-10-Alkylgruppe, Cycloalkylgruppe, Arylgruppe, Alkenylgruppe, Acylgruppe, Halogengruppe, Estergruppe, Aminogruppe, Amidgruppe, Carboxylgruppe, Hydroxylgruppe, Nitrogruppe, Cyangruppe, Sulfenylgruppe, Sulfogruppe oder Thiolgruppe substituiert werden können, worin ein oder mehrere Wasserstoffatome in jedem der genannten Substituenten weiter durch W substituiert werden können, A eine Amidgruppe mit einem Bindungsmodus "M-NR¹C(O)-E" oder ein Sauerstoffatom ist, worin A ein Sauerstoffatom ist, E eine Arylengruppe ist, in der ein oder mehrere Wasserstoffatome durch einen Substituenten W substituiert werden können, R¹ ein Wasserstoffatom, eine C1-10-Alkylgruppe, eine Cycloalkylgruppe, eine Arylgruppe, eine Heterocyclylgruppe, eine Alkenylgruppe, eine Alkynylgruppe, eine Estergruppe, eine Acylgruppe, eine Amidgruppe, eine Phosphingruppe, eine Sulfenylgruppe, eine Sulfinylgruppe, eine Sulfonylgruppe oder eine Silylgruppe bezeichnet, in dem oder der ein oder mehrere Wasserstoffatome durch einen Substituenten W substituiert werden können, worin ein oder mehrere Wasserstoffatome im Substituenten W weiter durch einen Substituenten W substituiert werden können, Y und Z identisch oder voneinander verschieden sind und eine C1-10-Alkylgruppe, eine Cycloalkylgruppe, eine Arylgruppe, eine Heterocyclylgruppe, eine Alkenylgruppe oder eine Silylgruppe bezeichnen, in der ein oder mehrere Wasserstoffatome durch einen Substituenten W substituiert werden können, wobei "Y und Z", "Z und E", "Y und E" oder "Y-Z-E" zur Bildung eines Rings gebunden sein können und n eine Ganzzahl von 0 oder 1 bezeichnet, worin bei n = 0 die Bindung zwischen N-Z oder N-E eine Doppelbindung ist.

2. Verfahren zur Herstellung einer Aminoverbindung gemäß Anspruch 1, **dadurch gekennzeichnet, dass** ein Teil der Formel (1) durch die nachfolgende allgemeine Formel (2) ausgedrückt wird: 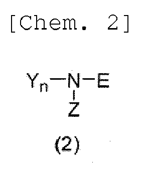 worin in der allgemeinen Formel (2), n = 0 und N, E und Z die gleiche Bedeutung wie definiert in Anspruch 1 haben, eine Gruppe ist, die eine stickstoffhaltige zyklische Gruppe umfasst.

3. Verfahren zur Herstellung einer Aminoverbindung gemäß Anspruch 1 oder 2, **dadurch gekennzeichnet, dass** die metallorganische Verbindung der Formel (1) eine Verbindung der allgemeinen Formel (3) ist: 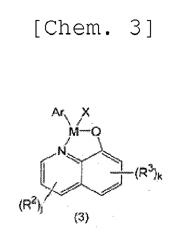 worin in der allgemeinen Formel (3) Ar, M und X die gleiche Bedeutung wie in Anspruch 1 haben, R² und R³ miteinander identisch oder voneinander verschieden sind und ein Wasserstoffatom, ein Halogenatom, eine Nitrogruppe, eine Cyangruppe, eine Hydroxylgruppe, eine Sulfogruppe, eine Mercaptogruppe, eine Carboxylgruppe oder die folgenden Gruppen bezeichnen, in denen ein oder mehrere Wasserstoffatome durch einen Substituenten W substituiert werden können: Eine C1-10-Alkylgruppe, eine Cycloalkylgruppe, eine Arylgruppe, eine Heterocyclylgruppe, eine Alkenylgruppe, eine Alkynylgruppe, eine Alkoxygruppe, eine Estergruppe, eine Fluoralkylgruppe, eine Acylgruppe, eine Sulfonylgruppe, eine Aminogruppe, eine Amidgruppe, eine Phosphingruppe, eine Sulfenylgruppe oder eine Silylgruppe, W die gleiche Bedeutung wie in Anspruch 1 beschrieben hat, j und k unabhängig voneinander eine Ganzzahl von 0 bis 3 sind, worin ein oder mehrere Kohlenstoffatome, die nicht an R² oder R³ gebunden sind, im Chinolinring durch ein Stickstoffatom substituiert werden können, und wenn j und/oder k1 oder mehr ist (sind), R² und R³ zur Bildung eines Rings miteinander verbunden werden können, und wenn j und/oder k 2 oder mehr ist (sind), zwei oder mehr von R² und/oder R³ zur Bildung eines Rings,
oder einer oder mehrerer metallorganischer Verbindungen, die aus der aus der metallorganischen Verbindung der nachfolgenden allgemeinen Formel (4) bestehenden Gruppe ausgewählt werden, miteinander verbunden werden können: 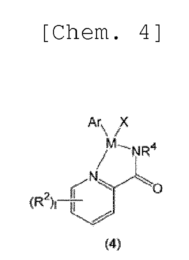 worin in der allgemeinen Formel (4) Ar, M und X die gleiche Bedeutung wie in Anspruch 1 haben, R² die gleiche Bedeutung wie die oben genannten R² und R³ hat, R⁴ ein Wasserstoffatom, eine C1-10-Alkylgruppe, eine Vycloalkylgruppe, eine Arylgruppe, eine Heterocyclylgruppe, eine Alkenylgruppe, eine Alkynylgruppe, eine Estergruppe, eine Acylgruppe, eine Amidgruppe, eine Phosphingruppe, eine Sulfenylgruppe, eine Sulfinylgruppe, eine Sulfonylgruppe oder eine Silylgruppe bezeichnet, in der ein oder mehrere Wasserstoffatome durch einen Substituenten W substituiert werden können, W die gleiche Bedeutung wie in Anspruch 1 beschrieben hat, 1 eine Ganzzahl von 0 bis 4 bezeichnet, worin ein oder mehrere Kohlenstoffatome im Pyridinring, die nicht an R² gebunden sind, durch ein Stickstoffatom substituiert werden können, und worin, wenn 1 2 oder mehr ist, zwei oder mehr von R² zur Bildung eines Rings miteinander verbunden sein können,
und die nachfolgende allgemeine Formel (5): 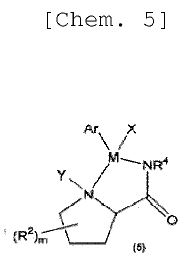 worin in der allgemeinen Formel (5) Ar, M, X und Y die gleiche Bedeutung wie in Anspruch 1 haben, R² die gleiche Bedeutung wie die oben genannten R² und R³ hat, R⁴ ein Wasserstoffatom, eine C1-10-Alkylgruppe, eine Cycloalkylgruppe, eine Arylgruppe, eine Heterocyclylgruppe, eine Alkenylgruppe, eine Alkynylgruppe, eine Estergruppe, eine Acylgruppe, eine Amidgruppe, eine Phosphingruppe, eine Sulfenylgruppe, eine Sulfinylgruppe, eine Sulfonylgruppe oder eine Silylgruppe bezeichnet, in der ein oder mehrere Wasserstoffatome durch einen Substituenten W substituiert werden können, W die gleiche Bedeutung wie in Anspruch 1 beschrieben hat, m eine Ganzzahl von 0 bis 7 bezeichnet, worin ein oder mehrere Kohlenstoffatome im Pyridinring durch ein Stickstoffatom substituiert werden können, und worin, wenn m 2 oder mehr ist, zwei oder mehr von R² zur Bildung eines Rings miteinander verbunden sein können.

4. Verfahren zur Herstellung einer Aminoverbindung, **dadurch gekennzeichnet, dass** eine Iminverbindung oder Enaminverbindung diastereoselektiv mit Wasserstoffgas oder einer organischen oder anorganischen Wasserstoff abgebenden Verbindung unter Vorhandensein einer metallorganischen Verbindung der folgenden allgemeinen Formel (6)reduziert wird: 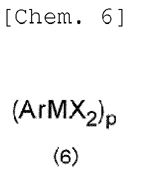 worin in der allgemeinen Formel (6) Ar, M und X die gleiche Bedeutung wie in Anspruch 1 definiert haben und p eine Ganzzahl von 2 oder mehr bezeichnet und eine organische Verbindung der allgemeinen Formel (7): 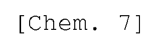 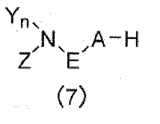 worin in der allgemeinen Formel (7) A, E, Y, Z und n die gleiche Bedeutung wie in Anspruch 1 definiert haben.

5. Verfahren zur Herstellung einer Aminoverbindung oder gemäß einem der Ansprüche 1 bis 4, **dadurch gekennzeichnet, dass** eine Carbonylverbindung mit einer Aminoverbindung oder einem Ammoniumsalz vermischt wird und die in diesem System erzeugte Iminverbindung oder Enaminverbindung diastereoselektiv reduziert wird.

6. Verfahren zur Herstellung einer Aminoverbindung gemäß einem der Ansprüche 1 bis 5, **dadurch gekennzeichnet, dass** M in der Formel (1) und der Formel (6) Rhodium oder Iridium ist.

7. Verfahren zur Herstellung einer Aminoverbindung gemäß einem der Ansprüche 1 bis 6, **dadurch gekennzeichnet, dass** die organische oder anorganische Wasserstoff abgebende Verbindung Ameisensäure oder Formiat ist.

8. Verfahren zur Herstellung einer Aminoverbindung gemäß einem der Ansprüche 1 bis 7, **dadurch gekennzeichnet, dass** die aus der Reaktion gewonnene Aminoverbindung eine zyklische Aminoverbindung oder eine zyklische Diaminverbindung ist.

## Revendications

1. Procédé de préparation d'un composé amine, **caractérisé en ce qu'**un composé imine ou un composé énamine est diastéréo-sélectivement réduit en utilisant un gaz hydrogène ou un composé donneur d'hydrogène organique ou inorganique, en présence d'un composé organométallique de formule générale (1) : 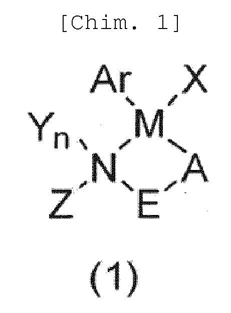 **caractérisé en ce que** dans la formule générale (1), Ar est un groupe cyclopentadiényle ou un composé aromatique, dans lequel un ou plusieurs atomes d'hydrogène peuvent être substitués par un substituant W, et W désigne un groupe hydrocarbure en C₁₋₁₀ saturé ou non saturé, un groupe aryle, un groupe hétérocyclyle, un groupe alcoxy, un groupe fluoroalkyle, un groupe acyle, un groupe ester, un groupe hydroxyle, un groupe amino, un groupe amide, un groupe carboxyle, un groupe sulfonyle, un groupe nitro, un groupe cyano, un groupe sulfényle, un groupe sulfo, un groupe mercapto, un groupe phosphino, un groupe silyle ou un groupe halogène, M est du ruthénium, du rhodium, ou de l'iridium, X est un groupe hydrure ou un groupe anionique, E désigne un groupe de liaison qui est un groupe hydrocarbure en C₁₋₆ saturé ou non saturé dans lequel un ou plusieurs atomes d'hydrogène peuvent être substitués par un substituant W, ou qui est un groupe arylène dans lequel un ou plusieurs atomes d'hydrogène peuvent être substitués par un groupe alkyle en C₁₋₁₀, un groupe cycloalkyle, un groupe aryle, un groupe alcényle, un groupe acyle, un groupe halogène, un groupe ester, un groupe amino, un groupe amide, un groupe carboxyle, un groupe hydroxyle, un groupe nitro, un groupe cyano, un groupe sulfényle, un groupe sulfo, ou un groupe thiol, **caractérisé en ce qu'**un ou plusieurs atomes d'hydrogène dans chacun desdits substituants peuvent en outre être substitués par W, A est un groupe amide ayant un mode de liaison de « M-NR¹C(O)-E », ou un atome d'oxygène, où lorsque A est un atome d'oxygène, E est un groupe arylène dans lequel un ou plusieurs atomes d'hydrogène peuvent être substitués par un substituant W, R¹ désigne un atome d'hydrogène, un groupe alkyle en C₁₋₁₀, un groupe cycloalkyle, un groupe aryle, un groupe hétérocyclyle, un groupe alcényle, un groupe alcynyle, un groupe ester, un groupe acyle, un groupe amide, un groupe phosphino, un groupe sulfényle, un groupe sulfinyle, un groupe sulfonyle ou un groupe silyle, dans lequel un ou plusieurs atomes d'hydrogène peuvent être substitués par un substituant W, **caractérisé en ce qu'**un ou plusieurs atomes d'hydrogène dans le substituant W peuvent en outre être substitués par un substituant W, Y et Z sont identiques ou différents l'un de l'autre, et désignent un groupe alkyle en C₁₋₁₀, un groupe cycloalkyle, un groupe aryle, un groupe hétérocyclyle, un groupe alcényle, ou un groupe silyle, dans lequel un ou plusieurs atomes d'hydrogène peuvent être substitués par un substituant W, où « Y et Z », « Z et E », « Y et E », ou « Y-Z-E » peuvent être liés pour former un cycle, et n désigne un entier de 0 ou 1, où, lorsque n = 0, la liaison entre N-Z ou N-E est une double liaison.

2. Procédé de préparation d'un composé amine selon la revendication 1, **caractérisé en ce qu'**une partie de formule (1) exprimée par la formule générale (2) ci-dessous : 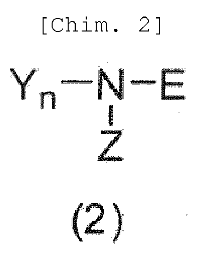 **caractérisé en ce que** dans la formule générale (2), n = 0 et N, E et Z ont les mêmes définitions que celles de la revendication 1, est un groupe comprenant un groupe cyclique contenant de l'azote.

3. Procédé de préparation d'un composé amine selon la revendication 1 ou 2, **caractérisé en ce que** le composé organométallique de formule (1) est un composé de formule générale (3) : 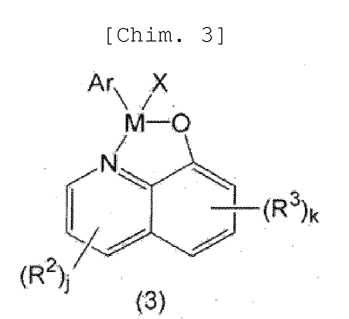 **caractérisé en ce que** dans la formule générale (3), Ar, M et X ont les mêmes définitions que celles décrites dans la revendication 1, R² et R³ sont identiques l'un à l'autre ou différents l'un de l'autre, et désignent un atome d'hydrogène, un atome d'halogène, un groupe nitro, un groupe cyano, un groupe hydroxyle, un groupe sulfo, un groupe mercapto, un groupe carboxyle, ou les groupes suivants dans lesquels un ou plusieurs atomes d'hydrogène peuvent être substitués par un substituant W : un groupe alkyle en C₁₋₁₀, un groupe cycloalkyle, un groupe aryle, un groupe hétérocyclyle, un groupe alcényle, un groupe alcynyle, un groupe alcoxy, un groupe ester, un groupe fluoroalkyle, un groupe acyle, un groupe sulfonyle, un groupe amino, un groupe amide, un groupe phosphino, un groupe sulfényle, ou un groupe silyle, W a la même définition que celle décrite dans la revendication 1, j et k sont, indépendamment l'un de l'autre, un entier de 0 à 3, où un ou plusieurs atomes de carbone, qui ne sont pas liés à R² ou R³, dans le cycle quinoléine peuvent être remplacés par un atome d'azote, et, lorsque j et/ou k est (sont) 1 ou plus, R² et R³ peuvent être liés l'un à l'autre pour former un cycle, et, lorsque j et/ou k est(sont) 2 ou plus, deux ou davantage de R² et/ou R³ peuvent être liés les uns aux autres pour former un cycle,
ou un ou plusieurs composés organométalliques choisis dans le groupe constitué du composé organométallique de formule générale (4) ci-dessous : 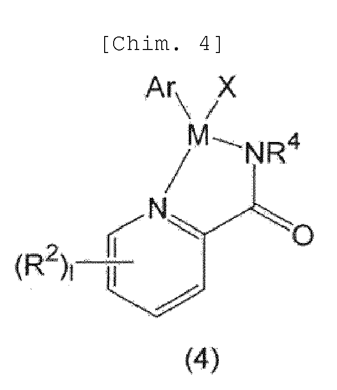 **caractérisé en ce que** dans la formule générale (4), Ar, M et X ont les mêmes définitions que celles décrites dans la revendication 1, R² a la même définition que les R² et R³ ci-dessus, R⁴ désigne un atome d'hydrogène, un groupe alkyle en C₁₋₁₀, un groupe cycloalkyle, un groupe aryle, un groupe hétérocyclyle, un groupe alcényle, un groupe alcynyle, un groupe ester, un groupe acyle, un groupe amide, un groupe phosphino, un groupe sulfényle, un groupe sulfinyle, un groupe sulfonyle ou un groupe silyle, dans lequel un ou plusieurs atomes d'hydrogène peuvent être substitués par un substituant W, W a la même définition que celle décrite dans la revendication 1, 1 désigne un entier de 0 à 4, où un ou plusieurs atomes de carbone, qui ne sont pas liés à R², dans le cycle pyridine peuvent être remplacés par un atome d'azote, et où, lorsque 1 est 2 ou davantage, deux ou davantage de R² peuvent être liés les uns aux autres pour former un cycle,
et de formule générale (5) ci-dessous : 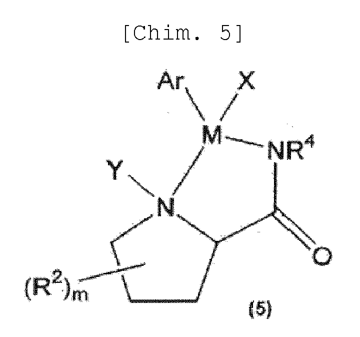 **caractérisé en ce que** dans la formule générale (5), Ar, M, X et Y ont les mêmes définitions que celles décrites dans la revendication 1, R² a la même définition que les R² et R³ ci-dessus, R⁴ désigne un atome d'hydrogène, un groupe alkyle en C₁₋₁₀, un groupe cycloalkyle, un groupe aryle, un groupe hétérocyclyle, un groupe alcényle, un groupe alcynyle, un groupe ester, un groupe acyle, un groupe amide, un groupe phosphino, un groupe sulfényle, un groupe sulfinyle, un groupe sulfonyle ou un groupe silyle, dans lequel un ou plusieurs atomes d'hydrogène peuvent être substitués par un substituant W, W a la même définition que celle décrite dans la revendication 1, m désigne un entier de 0 à 7, où un ou plusieurs atomes de carbone dans le cycle pyrrolidine peuvent être remplacés par un atome d'azote, et où, lorsque m est 2 ou davantage, deux ou davantage de R² peuvent être liés les uns aux autres pour former un cycle.

4. Procédé de préparation d'un composé amine, **caractérisé en ce qu'**un composé imine ou un composé énamine est diastéréo-sélectivement réduit en utilisant un gaz hydrogène ou un composé donneur d'hydrogène organique ou inorganique, en présence d'un composé organométallique de formule générale (6) : 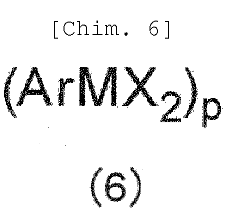 **caractérisé en ce que** dans la formule générale (6), Ar, M et X ont les mêmes définitions que celles décrites dans la revendication 1, et p désigne un entier de 2 ou davantage, et un composé organique de formule générale (7) : 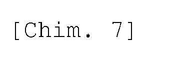 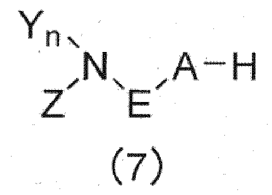 **caractérisé en ce que** dans la formule générale (7), A, E, Y, Z et n ont les mêmes définitions que celles décrites dans la revendication 1.

5. Procédé de préparation d'un composé amine ou selon l'une quelconque des revendications 1 à 4, **caractérisé en ce qu'**un composé carbonyle est mélangé avec un composé amine ou un sel d'ammonium, et le composé imine ou le composé énamine généré dans ce système est diastéréo-sélectivement réduit.

6. Procédé de préparation d'un composé amine selon l'une quelconque des revendications 1 à 5, **caractérisé en ce que** M dans la formule (1) et la formule (6) est du rhodium ou de l'iridium.

7. Procédé de préparation d'un composé amine selon l'une quelconque des revendications 1 à 6, **caractérisé en ce que** le composé donneur d'hydrogène organique ou inorganique est l'acide formique ou le formiate.

8. Procédé de préparation d'un composé amine selon l'une quelconque des revendications 1 à 7, **caractérisé en ce que** le composé amine obtenu à partir de la réaction est un composé amine cyclique ou un composé diamine cyclique.
